# EUROPEAN PATENT SPECIFICATION

(11) **EP 4 483 885 B1**
(45) Date of publication and mention of the grant of the patent: **24.12.2025**
(21) Application number: 23784278.6
(22) Date of filing: 04.04.2023
(51) Int. Cl.: A61K 9/16, A61K 9/48, A61K 31/53, A61K 9/14, A61K 9/20, A61P 31/14, A61P 11/00

(54) **COMPOSITION, METHOD FOR PREPARING SAME, AND USE THEREOF**
ZUSAMMENSETZUNG, VERFAHREN ZUR HERSTELLUNG DAVON UND VERWENDUNG DAVON
COMPOSITION, SON PROCÉDÉ DE PRÉPARATION ET SON UTILISATION

(30) Priority: 06.04.2022 CN 202210354088
(43) Date of publication of application: 01.01.2025
(73) Proprietor: Shenzhen Antiv Pharma Co., Ltd., Shenzhen, Guangdong 518000 (CN)
(72) Inventor: LI, Shuo, Shenzhen, Guangdong 518000 (CN); LI, Guanguan, Shenzhen, Guangdong 518000 (CN); LIU, Xinjun, Shenzhen, Guangdong 518000 (CN)
(74) Representative: Chung, Hoi Kan
(86) International application number: PCT/CN2023/086224
(87) International publication number: WO 2023/193715

(56) References cited:
- WO-A1-2021/240543
- CN-A- 113 185 519
- CN-A- 113 735 862
- CN-A- 113 735 862
- US-A1- 2022 081 462

## Description

### TECHNICAL FIELD

The present invention relates to the field of pharmaceutical formulations, in particular to a composition, a preparation method therefor, and use thereof.

### BACKGROUND

The novel coronavirus is an enveloped single-stranded RNA virus belonging to the β-genus coronavirus. Similar to SARS and MERS, the SARS-CoV-2 genome encodes non-structural proteins: 3-chymotrypsin-like protease (3CLpro), papain-like protease (PLpro), helicase, and RNA-dependent RNA polymerase (RdRp); structural proteins: such as spike glycoprotein and accessory proteins. The surface spike glycoprotein of the novel coronavirus binds to the angiotensin-converting enzyme (ACE2) receptor on the surface of human cells, thereby infecting the epithelial cells of the human respiratory tract. After the virus enters the host cell, it disassembles and releases the nucleocapsid and viral RNA into the cytoplasm. The 5' end open reading frame (ORF1a/b) of the viral RNA encodes polyproteins (pp1a and pp1ab), which play a crucial role in the processing and maturation of enzymes required for viral replication. pp1a and pp1ab can be cleaved by papain-like protease (PLpro) and 3-chymotrypsin-like protease (3CLpro) to produce non-structural proteins, including RNA-dependent RNA polymerase, helicase, etc., which are critical for the transcription and replication of the novel coronavirus. Currently, the surface spike glycoprotein of the novel coronavirus that recognizes the receptor, and the key proteins involved in the replication and transcription processes-3CLpro, PLpro, and RdRp-are four highly attractive targets for antiviral drug development.

Regarding the development of novel coronavirus vaccines, on Dec. 2, the UK first approved emergency use of Pfizer and BioNTech novel coronavirus vaccines. On one hand, the general use effect of the vaccine is not known. On the other hand, the strict low-temperature storage requirement brings great inconvenience to the wide use of the vaccine.

Regarding novel coronavirus drug development, Remdesivir is currently the only approved novel coronavirus drug by the FDA in the United States. Remdesivir is an aminomethyl monophosphate prodrug of an adenosine analog, originally developed by Gilead as an anti-Ebola virus drug. Remdesivir, as an RdRp inhibitor, shows the activity against novel coronavirus at a cell level, but clinical tests show that Remdesivir does not significantly reduce the mortality on a human body. Moreover, some significant side effects have to be noted since the clinically used dose is close to the safe dose.

Therefore, there is still a need for a drug for treating novel coronavirus with good safety, stability, and bioavailability.

### SUMMARY

### Brief Summary

In order to solve the problems described above, the present invention provides a composition comprising a compound ATV014 and a pharmaceutically acceptable excipient,

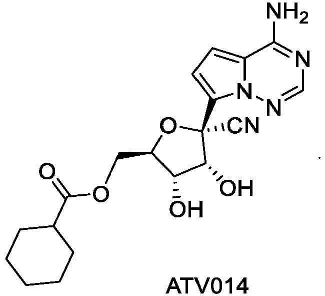

The pharmaceutically acceptable excipient comprises at least one selected from a diluent, a disintegrant, a binder, and a lubricant, it does not comprise a surfactant, and it may comprise other auxiliary materials. In some embodiments, the diluent is at least one of microcrystalline cellulose, mannitol, and pregelatinized starch. In some embodiments, the diluent is microcrystalline cellulose and mannitol. In some embodiments, the diluent is microcrystalline cellulose or mannitol. Using microcrystalline cellulose or mannitol as the diluent is more beneficial for improving the compatibility of raw material and auxiliary material, and microcrystalline cellulose or mannitol is preferably used as the diluent; more preferably, microcrystalline cellulose PH102 or mannitol 50C is used as the diluent; most preferably, mannitol 50C is used as the diluent.

In a second aspect, the present invention provides use of the aforementioned composition in the preparation of a product for preventing, alleviating or treating a coronavirus infection, or replication or propagation of a homologous variant virus thereof, and a cytopathic effect generated therefrom.

In a third aspect, the present invention provides a method for preparing the aforementioned composition by grinding or pulverizing ATV014 to reduce the particle size of ATV014, which is beneficial for increasing the dissolution rate of the product and for moderating the particle hardness during dry granulation. The preparation method adopts the ground ATV014 for dry granulation, which is beneficial for giving particles with relatively good hardness. The preparation method adopts dry granulation, which is beneficial for improving the disintegration rate of the product.

### Detailed Description of the Invention

In order to solve the problems described above, the present invention provides a composition and a pharmaceutical formulation thereof, use, and a preparation method.

In a first aspect, a composition is provided.

A composition, comprising: a compound ATV014 and a pharmaceutically acceptable excipient,

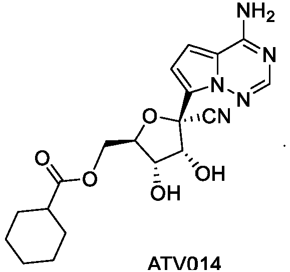

the pharmaceutically acceptable excipient comprises a diluent, a disintegrant, a binder, and a lubricant.

In some embodiments, the diluent may comprise at least one selected from microcrystalline cellulose, mannitol, calcium hydrophosphate, and pregelatinized starch. In some embodiments, the diluent is at least one of microcrystalline cellulose, mannitol, and pregelatinized starch. In some embodiments, the diluent is microcrystalline cellulose and mannitol. In some embodiments, the diluent is microcrystalline cellulose or mannitol. Microcrystalline cellulose or mannitol is adopted as the diluent, which is beneficial for improving the compatibility of raw material and auxiliary material. In some more preferred embodiments, the dilution is microcrystalline cellulose PH102 or mannitol 50C, which is more beneficial for improving the compatibility of raw material and auxiliary material. In some most preferred embodiments, the diluent is mannitol 50C, which is most beneficial for improving the compatibility of raw material and auxiliary material.

In some embodiments, the disintegrant may comprise at least one selected from croscarmellose sodium, crospovidone, sodium starch glycolate, and hydroxypropyl cellulose. In some embodiments, the disintegrant is croscarmellose sodium.

In some embodiments, the binder may comprise at least one selected from hydroxypropyl cellulose, povidone, and starch. In some embodiments, the binder is hydroxypropyl cellulose.

In some embodiments, the lubricant may comprise at least one selected from magnesium stearate, stearic acid, and sodium stearyl fumarate. In some embodiments, the lubricant is magnesium stearate.

In some embodiments, the other auxiliary materials may comprise an auxiliary material selected from a glidant.

In some embodiments, the glidant may comprise a glidant selected from colloidal silicon dioxide or

In some embodiments, the content of the compound ATV014 may be 15 wt% to 70 wt%, based on the total mass of the composition. In some embodiments, the content of the compound ATV014 is 50 wt% to 60 wt%, based on the total mass of the composition. In some embodiments, the content of the compound ATV014 is 15 wt%, 20 wt%, 25 wt%, 30 wt%, 35 wt%, 40 wt%, 45 wt%, 50 wt%, 55 wt%, 56 wt%, 60 wt%, 65 wt%, or 70 wt%, based on the total mass of the composition.

In some embodiments, the content of the diluent may be 20 wt% to 70 wt%, based on the total mass of the composition. In some embodiments, the content of the diluent is 30 wt% to 40 wt%, based on the total mass of the composition. In some embodiments, the content of the diluent is 20 wt%, 25 wt%, 30 wt%, 35 wt%, 36 wt%, 37 wt%, 40 wt%, 45 wt%, 50 wt%, 55 wt%, 60 wt%, 65 wt%, or 70 wt%, based on the total mass of the composition.

In some embodiments, the content of the disintegrant may be 1 wt% to 10 wt%, based on the total mass of the composition. In some embodiments, the content of the disintegrant is 2 wt% to 5 wt%, based on the total mass of the composition. In some embodiments, the content of the disintegrant is 1 wt%, 2 wt%, 3 wt%, 4 wt%, 5 wt%, 6 wt%, 7 wt%, 8 wt%, 9 wt%, or 10 wt%, based on the total mass of the composition.

In some embodiments, the content of the binder may be 1 wt% to 10 wt%, based on the total mass of the composition. In some embodiments, the content of the binder is 2 wt% to 5 wt%, based on the total mass of the composition. In some embodiments, the content of the binder is 1 wt%, 2 wt%, 3 wt%, 4 wt%, 5 wt%, 6 wt%, 7 wt%, 8 wt%, 9 wt%, or 10 wt%, based on the total mass of the composition.

In some embodiments, the content of the lubricant may be 0.5 wt% to 5 wt%, based on the total mass of the composition. In some embodiments, the content of the lubricant is 1.5 wt% to 5 wt%, based on the total mass of the composition. In some embodiments, the content of the lubricant is 0.5 wt%, 1 wt%, 1.5 wt%, 2 wt%, 3 wt%, 4 wt%, or 5 wt%, based on the total mass of the composition.

The composition does not comprise a surfactant. In some embodiments, the composition does not comprise sodium lauryl sulfate.

In some embodiments, the composition may further comprise an external lubricant.

In some embodiments, the external lubricant comprises at least one selected from magnesium stearate, sodium stearate, and sodium stearyl fumarate. In some embodiments, the external lubricant is magnesium stearate.

In some embodiments, the content of the external lubricant is 0.5 wt% to 5 wt%, based on the total mass of the composition. In some embodiments, the content of the external lubricant is 1.5 wt% to 5 wt%, based on the total mass of the composition. In some embodiments, the content of the external lubricant is 0.5 wt%, 1 wt%, 1.5 wt%, 2 wt%, 3 wt%, 4 wt%, or 5 wt%, based on the total mass of the composition.

In some embodiments of the present invention, a composition, comprising: a compound ATV014 and a pharmaceutically acceptable excipient, the pharmaceutically acceptable excipient comprising at least one selected from a diluent, a disintegrant, a binder, a lubricant, and other auxiliary materials, the diluent comprising at least one selected from microcrystalline cellulose, mannitol, calcium hydrophosphate, and pregelatinized starch, the disintegrant comprising at least one selected from croscarmellose sodium, crospovidone, sodium starch glycolate, and hydroxypropyl cellulose, the binder comprising at least one selected from hydroxypropyl cellulose, povidone, and starch, and the lubricant comprising at least one selected from magnesium stearate, stearic acid, and sodium stearyl fumarate, wherein based on the total mass of the composition, the content of the compound ATV014 is 15 wt% to 70 wt%, the content of the diluent is 20 wt% to 70 wt%, the content of the disintegrant is 1 wt% to 10 wt%, the content of the binder is 1 wt% to 10 wt%, and the content of the lubricant may be 0.5 wt% to 5 wt%.

In some embodiments of the present invention, a composition, comprising: a compound ATV014 and a pharmaceutically acceptable excipient, the pharmaceutically acceptable excipient comprising at least one selected from a diluent, a disintegrant, a binder, a lubricant, and other auxiliary materials, the diluent comprising at least one selected from microcrystalline cellulose, mannitol, calcium hydrophosphate, and pregelatinized starch, the disintegrant comprising at least one selected from croscarmellose sodium, crospovidone, sodium starch glycolate, and hydroxypropyl cellulose, the binder comprising at least one selected from hydroxypropyl cellulose, povidone, and starch, and the lubricant comprising at least one selected from magnesium stearate, stearic acid, and sodium stearyl fumarate, wherein based on the total mass of the composition, the content of the compound ATV014 is 50 wt% to 60 wt%, the content of the diluent is 30 wt% to 40 wt%, the content of the disintegrant is 2 wt% to 5 wt%, the content of the binder is 2 wt% to 5 wt%, and the content of the lubricant may be 1.5% to 5 wt%.

In some embodiments of the present invention, a composition, comprising: a compound ATV014 and a pharmaceutically acceptable excipient, the pharmaceutically acceptable excipient comprising at least one selected from a diluent, a disintegrant, a binder, a lubricant, and other auxiliary materials, the diluent comprising at least one selected from microcrystalline cellulose and mannitol, the disintegrant comprising a disintegrant selected from croscarmellose sodium, the binder comprising a binder selected from hydroxypropyl cellulose, and the lubricant comprising at least one selected from magnesium stearate, stearic acid, and sodium stearyl fumarate, wherein based on the total mass of the composition, the content of the compound ATV014 is 50 wt% to 60 wt%, the content of the diluent is 30 wt% to 40 wt%, the content of the disintegrant is 2 wt% to 5 wt%, the content of the binder is 2 wt% to 5 wt%, and the content of the lubricant may be 1.5% to 5 wt%.

In some embodiments of the present invention, a composition, comprising: a compound ATV014 and a pharmaceutically acceptable excipient, the pharmaceutically acceptable excipient comprising at least one selected from a diluent, a disintegrant, a binder, a lubricant, and other auxiliary materials, the diluent being microcrystalline cellulose PH102 or mannitol 50C, the disintegrant comprising a disintegrant selected from croscarmellose sodium, the binder comprising a binder selected from hydroxypropyl cellulose, and the lubricant comprising at least one selected from magnesium stearate, stearic acid, and sodium stearyl fumarate, wherein based on the total mass of the composition, the content of the compound ATV014 is 50 wt% to 60 wt%, the content of the diluent is 30 wt% to 40 wt%, the content of the disintegrant is 2 wt% to 5 wt%, the content of the binder is 2 wt% to 5 wt%, and the content of the lubricant may be 1.5% to 5 wt%.

In some embodiments of the present invention, a composition, comprising: a compound ATV014 and a pharmaceutically acceptable excipient, the pharmaceutically acceptable excipient comprising at least one selected from a diluent, a disintegrant, a binder, a lubricant, and other auxiliary materials, the diluent being microcrystalline cellulose PH102 and mannitol 50C, the disintegrant comprising a disintegrant selected from croscarmellose sodium, the binder comprising a binder selected from hydroxypropyl cellulose, and the lubricant comprising at least one selected from magnesium stearate, stearic acid, and sodium stearyl fumarate, wherein based on the total mass of the composition, the content of the compound ATV014 is 50 wt% to 60 wt%, the content of the diluent is 30 wt% to 40 wt%, the content of the disintegrant is 2 wt% to 5 wt%, the content of the binder is 2 wt% to 5 wt%, and the content of the lubricant may be 1.5% to 5 wt%.

In some embodiments of the present invention, a composition, comprising: a compound ATV014 and a pharmaceutically acceptable excipient, the pharmaceutically acceptable excipient comprising at least one selected from a diluent, a disintegrant, a binder, a lubricant, and other auxiliary materials, the diluent being mannitol 50C, the disintegrant comprising a disintegrant selected from croscarmellose sodium, the binder comprising a binder selected from hydroxypropyl cellulose, and the lubricant comprising at least one selected from magnesium stearate, stearic acid, and sodium stearyl fumarate, wherein based on the total mass of the composition, the content of the compound ATV014 is 50 wt% to 60 wt%, the content of the diluent is 30 wt% to 40 wt%, the content of the disintegrant is 2 wt% to 5 wt%, the content of the binder is 2 wt% to 5 wt%, and the content of the lubricant may be 1.5% to 5 wt%.

In some embodiments, the dosage form of the composition may be a solid oral dosage form or an injection. In some embodiments, the dosage form of the composition is a tablet, a granule, or a capsule.

In some embodiments, the specification of the dosage form may be 10 mg to 500 mg (e.g., 10 mg, 15 mg, 20 mg, 25 mg, 30 mg, 35 mg, 40 mg, 45 mg, 50 mg, 100 mg, 150 mg, 200 mg, 250 mg, 300 mg, 350 mg, 400 mg, 450 mg, or 500 mg). In some embodiments, the specification of the dosage form is 15 mg to 300 mg. In some embodiments, the specification of the dosage form is 20 mg to 200 mg. In some embodiments, the specification of the dosage form is 50 mg to 200 mg. In some embodiments, the specification of the dosage form is 50 mg.

In a third aspect, use is provided.

Use of the composition according to the first aspect in the preparation of a drug.

In some embodiments, use of the composition according to the first aspect in the preparation of a product for preventing, alleviating or treating a coronavirus infection, or replication or propagation of a homologous variant virus thereof, and a cytopathic effect generated therefrom.

In some embodiments, the infection comprises fever, cough, sore throat, pneumonia, acute respiratory infection, severe acute respiratory infection, hypoxic respiratory failure and acute respiratory distress syndrome, sepsis, or septic shock.

In some embodiments, use of the composition according to the first aspect in the preparation of a product for detecting a coronavirus or a homologous variant virus thereof.

In some embodiments, the coronavirus comprises: MHV-A59, HCoV-229E, HCoV-OC43, HCoV-NL63, HCoV-HKU1, SARS-CoV, MERS-CoV, SARS-CoV-2, a mouse hepatitis virus, a feline infectious peritonitis virus, a canine coronavirus, a bovine coronavirus, an avian infectious bronchitis virus, or a porcine coronavirus; preferably, the SARS-CoV-2 comprises a mutant strain or a non-mutant strain of SARS-CoV-2; more preferably, the mutant strain of SARS-CoV-2 comprises SARS-CoV-2 mutant strain B.1, SARS-CoV-2 mutant strain B.1.351, SARS-CoV-2 mutant strain B.1.617.2, SARS-CoV-2 mutant strain C.37, SARS-CoV-2 mutant strain P.1 lineage, SARS-CoV-2 mutant strain B.1.525, SARS-CoV-2 mutant strain B.1.427, or SARS-CoV-2 mutant strain B.1.429.

In some embodiments, the composition is suitable for use in humans or animals. In some embodiments, the animal comprises bovine, equine, ovine, porcine, canine, feline, rodent, primate, avian, or piscine animal.

In a fourth aspect, a method for preparing the composition according to the first aspect is provided.

A method for preparing the composition according to the first aspect, comprising: mixing the compound ATV014 and the pharmaceutically acceptable excipient, performing dry granulation, adding an external lubricant, mixing, and tableting or capsule filling or packaging to give the composition.

In some embodiments of the present invention, a method for preparing the composition according to the first aspect, comprising: mixing the compound ATV014 and the pharmaceutically acceptable excipient, performing wet granulation, grinding, drying, dry grinding, adding an external lubricant, mixing, and tableting or capsule filling or packaging to give the composition.

In some embodiments of the present invention, a method for preparing the composition according to the first aspect, comprising: grinding or pulverizing the compound ATV014, mixing the compound and the pharmaceutically acceptable excipient, performing dry granulation, adding an external lubricant, mixing, and tableting or capsule filling or packaging to give the composition.

In some embodiments of the present invention, a method for preparing the composition according to the first aspect, comprising: grinding or pulverizing the compound ATV014, mixing the compound and the pharmaceutically acceptable excipient, performing wet granulation, grinding, drying, dry grinding, adding an external lubricant, mixing, and tableting or capsule filling or packaging to give the composition.

In some embodiments, the particle size D90 of the ATV014 may be ≤ 500 µm. In some embodiments, the particle size D90 of the ATV014 is ≤ 300 µm. In some embodiments, the particle size D90 of the ATV014 is ≤ 200 µm. In some embodiments, the particle size D90 of the ATV014 is ≤ 100 µm. In some embodiments, the particle size D90 of the ATV014 is ≤ 50 µm. In some preferred embodiments, the particle size D90 of the ATV014 is ≤ 25 µm. In some preferred embodiments, the particle size D90 of the ATV014 is ≤ 20 µm. In some more preferred embodiments, the particle size D90 of the ATV014 is ≤ 10 µm.

### Beneficial Effects

Compared with the prior art, one embodiment of the present invention at least has one of the following beneficial technical effects:
(1) Using the formula and the formula proportion adopted by the present invention is beneficial for improving the dissolution and the bioavailability.
(2) Using the formula and the formula proportion adopted by the present invention is beneficial for improving the stability of the drug under the conditions of high temperature, high humidity, illumination, and the like.
(3) Using the formula and the formula proportion adopted by the present invention can be used for preventing, alleviating or treating a coronavirus infection, or replication or propagation of a homologous variant virus thereof, and a cytopathic effect generated therefrom.
(4) Using the mode of not adding the surfactant is beneficial for improving the dissolution rate of the product.
(5) The content of the lubricant (e.g., magnesium stearate) is preferably 1.5% or more, which is beneficial for the hardness and smoothness of the dry granulated particles.
(6) The content of the binder (e.g., hydroxypropyl cellulose) is preferably 3% or more, which is beneficial for the hardness and smoothness of the dry granulated particles.
(7) The content of the binder (e.g., hydroxypropyl cellulose) is preferably about 3%, which is beneficial for the dissolution of the product.
(8) Using a method of grinding or pulverizing ATV014 to reduce the particle size of ATV014 is beneficial for increasing the dissolution rate of the product and for moderating the particle hardness during dry granulation.
(9) Using the ground ATV014 for dry granulation is beneficial for giving particles with relatively good hardness.
(10) Using dry granulation is beneficial for improving the disintegration rate of the product.
(11) Compared with other diluents, using microcrystalline cellulose or mannitol as the diluent is more beneficial for improving the compatibility of raw material and auxiliary material, and microcrystalline cellulose or mannitol is preferably used as the diluent, more preferably, microcrystalline cellulose PH102 or mannitol 50C, and most preferably, mannitol 50C.

### Definitions of Terms:

In the present invention, "room temperature" presents ambient temperature, and may be 20 °C to 30 °C; in some embodiments, the temperature is 22 °C to 28 °C; in some embodiments, the temperature is 24 °C to 26 °C; and in some embodiments, the temperature is 25 °C.

In the context of the present invention, all numbers disclosed herein are approximate values, whether or not the word "about" or "approximately" is used. Based on the disclosed numbers, it is possible that the numerical value of each number may have a difference of ±10% or less or a reasonable difference considered by those skilled in the art, such as by ±1%, ±2%, ±3%, ±4%, or ±5%.

The term "optional" or "optionally" means that the subsequently described event or case may or may not occur. For example, "optional surfactant" means that the surfactant may or may not be present.

The term "external lubricant" refers to a substance added after granulation to reduce friction between the resulting particles from granulation, to prevent the raw material and the auxiliary material from sticking to the punch surface or to reduce friction between the tablet and the well wall of the punch die.

The term "specification" refers to the weight of the active ingredient in one unit of formulation (single tablet or single capsule).

The term "weight percentage" or "percentage by weight" or "wt%" is defined as follows: The weight of an individual component in a composition is divided by the total weight of all components in the composition and multiplied by 100.

The term "and/or" should be understood to refer to any one of the options or a combination of any two or more of the options.

The term "wt%" means mass percentage.

As used herein, the term "treating" refers to a clinical intervention intending to alter the natural progress of a disease in an individual being treated. Desired therapeutic effects include, but are not limited to, preventing the occurrence or recurrence of diseases, alleviating symptoms, reducing any direct or indirect pathological outcomes of diseases, preventing metastasis, delaying disease progression, improving or alleviating conditions, and alleviating or improving prognosis.

In the specification, terms such as "one embodiment", "some embodiments", "examples", "a specific example", or "some examples", mean that a particular feature, structure, material, or characteristic described in reference to the embodiment or example is included in at least one embodiment or example of the present invention. In this specification, the schematic descriptions of the terms described above do not necessarily refer to the same embodiment or example. Moreover, the specific features, materials, structures, and characteristics described may be combined in any one or more embodiments or examples in an appropriate manner. Moreover, various embodiments or examples and features of various embodiments or examples described in this specification can be combined by one skilled in the art to the extent that they do not contradict each other.

In the present application, a "composition" may be conveniently presented in unit dose form and may be prepared by any of the methods well known in the pharmaceutical art. All the methods include the step of combining the active ingredient with the carrier which constitutes one or more accessory ingredients. Generally, compositions are prepared by uniformly and sufficiently combining the active compound with a liquid carrier, a finely divided solid carrier, or both.

In the present invention, the structures of some compounds represented by abbreviations are shown.

| **Abbreviation** | **Chemical structure** |
|---|---|
| GS-441524 | 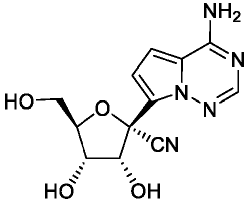 |
| ATV014 intermediate **5** | 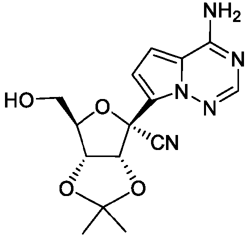 |

### DETAILED DESCRIPTION OF THE EMBODIMENTS

In order to make the technical solutions of the present invention better understood by those skilled in the art, some non-limiting examples are further disclosed below to further explain the present invention in detail.

The reagents used in the present invention are either commercially available or can be prepared by the methods described herein.

"#0" indicates capsule shell #0. "#4" indicates capsule shell #4. "API" refers to a drug substance and in the following specific examples refers to the compound ATV014. "rpm" refers to the unit of rotational speed "revolutions per minute". "SDS" refers to sodium dodecyl sulfonate. "h" refers to hour(s). "min" refers to minute(s). "µM" refers to micromole(s) per liter, i.e., µmol/L. "mL" or "ml" refers to milliliter(s). "Compound RDV" or "RDV" refers to Remdesivir.

### Example 1: Preparation of ATV014

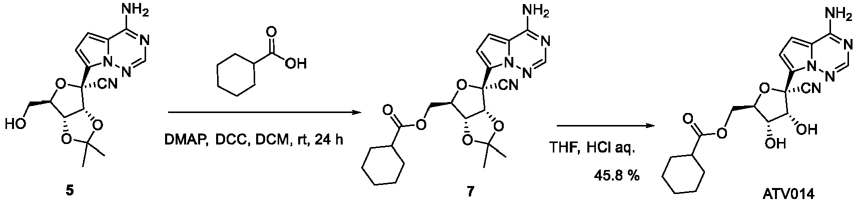

1.50 g of compound **5** was dissolved in 15 ml of dichloromethane, then 0.42 mL of cyclohexyl carboxylic acid and 55.40 mg of 4-dimethylaminopyridine were added. After stirring for 10 min, 1.02 g of dicyclohexylcarbodiimide was added, and the mixture was stirred at room temperature for 24 h. The mixture was separated by column chromatography (eluent: petroleum ether/ethyl acetate (V/V) = 1/1) to give Compound **7.**

1.50 g of compound **7** was dissolved in 3 mL of hydrochloric acid aqueous solution with a mass percentage of 37% and 15 mL of tetrahydrofuran. After stirring for 6 h, sodium carbonate was added to adjust the pH to 8. The organic solvent was removed by rotary evaporation. The mixture was separated by column chromatography (eluent: petroleum ether/ethyl acetate (V/V) = 1/3) to give 0.28 g of compound ATV014 (white solid, yield: 45.8%). The compound ATV014 obtained was measured for hydrogen spectrum and carbon spectrum, and the results were as follows:

Hydrogen spectrum: ¹H NMR (600 MHz, DMSO-*d₆*) δ (ppm): 7.92 (s, 1H), 7.86 (br, 1H), 6.92 (d, *J*=4.5 Hz,1H), 6.81 (d, *J=*4.5 Hz, 1H), 6.33 (d, *J*=5.9 Hz, 1H), 5.38 (d, *J*=5.9 Hz, 1H), 4.70 (t, *J*=5.3 Hz, 1H), 4.32-4.29 (dd, *J*=12.2 Hz, 2.6 Hz, 1H), 4.24-4.21 (m, 1H), 4.16-4.13 (dd, *J*=12.3 Hz, 4.8 Hz, 1H), 3.98-3.95 (q, *J*=5.9 Hz, 1H), 2.26-2.22 (m, 1H), 1.75-1.72 (m, 2H), 1.64-1.56 (m, 3H), 1.30-1.12 (m, 5H).

Carbon spectrum: ¹³C NMR (150 MHz, DMSO-*d₆*) δ (ppm): 175.34, 156.06, 148.4, 124.0, 117.4, 117.0, 110.7, 101.2, 81.7, 79.4, 74.5, 70.6, 63.0, 42.6, 29.0, 28.9, 25.7, 25.2, 25.1.

### Example 2: Inhibitory Effect of Compounds on SARS-CoV-2 Replicon on HEK293T Cell

The compound GS-441524, ATV014, or ATV014 intermediate 5 was separately taken as the test compound, and the following steps were separately performed.

HEK293T cells were inoculated onto a 24-well plate. When the cells grew to a density of 40-50%, the cells were transfected with 250 ng of a SARS-CoV-2 replicon plasmid by LIPO2000 (liposome 2000). After transfecting for 6-8 h, the cell supernatant was discarded, the medium was replaced with a fresh DMEM medium, and the test compound was separately added until the final concentration was 50 µM, 10 µM, 5 µM, 2 µM, 1 µM, 0.1 µM, or 0.01 µM. After transfecting for 60 h, the cell supernatant was discarded, the cell RNA was collected by TRIZOL. The cDNA was obtained by reverse transcription of extracted total RNA. Finally, the subgenome of the reference gene Gapdh and SARS-CoV-2 N gene in the cDNA was detected by fluorescence quantitative PCR to reflect the virus replication in the SARS-CoV-2 replicon. The inhibitory effect of drugs with different concentrations on the virus was calculated, and IC₅₀ of the drugs was calculated. The results are shown in Table 2.

**Table 2: Inhibitory effect of compound on SARS-CoV-2 replicon on HEK293T cell**

| **Compound** | **Inhibition rate% (10 µM)** | ***IC₅₀ (µM)*** |
|---|---|---|
| GS-441524 | 99.79 | 0.95 |
| ATV014 | 98.23 | 0.26 |
| ATV014 intermediate 5 | NA | > 50 |
| Note | NA indicates no data. | |

**Conclusion:** The test compounds inhibited the replication of SARS-CoV-2 to varying degrees in HEK293T cells. The activity of ATV014 was 3.65 times of that of compound GS-441524, and the activity was remarkably improved. Inhibitory effect of different compounds on the SARS-CoV-2 replicon on HEK293T cells is shown in Table 2.

### Example 3: Inhibition Effect of Compounds on SARS-CoV-2 in Vero-E6 Cell

Compounds RDV, GS-441524, and ATV014 were separately taken as the test compounds, and the following steps were performed.

Vero-E6 cells were inoculated onto a 48-well plate. When the cell density was about 70-80%, the supernatant was discarded, and the medium was replaced with a fresh DMEM medium. Each compound was then separately added to the medium such that the final concentration of the compound was 50 µM, 10 µM, 5 µM, 2 µM, 1 µM, 0.5 µM, 0.25 µM, 0.1 µM, or 0.01 µM. The cells infected three SARS-CoV-2 mutants (B.1, B.1.351, and B.1.617.2) at a multiplicity of infection (MOI) of 0.05. The antiviral activity and viral copies in supernatant 48 h after quantitative infection were evaluated by quantitative real-time polymerase chain reaction (qRT-PCR). We calculated the inhibitory effect of the test drugs at different concentrations on virus replication and calculated their IC50 values. The IC50 of the different compounds in Vero-E6 cells for SARS-CoV-2 is shown in Table 3.

**Table 3: Inhibition effect of different compounds on SARS-CoV-2 in Vero-E6 cell**

| **Compound** | **IC₅₀ (µM)** | | |
|---|---|---|---|
| | SARS-CoV-2 mutant strain B. 1 | SARS-CoV-2 mutant strain B.1.351 | SARS-CoV-2 mutant strain B.1.617.2 |
| GS-441524 | 2.279 | 1.780 | 1.645 |
| RDV | 1.709 | 1.354 | 0.9573 |
| ATV014 | 0.3313 | 0.2484 | 0.2097 |

Example 4: Investigation of Surfactant (Preparation Method being Wet Granulation)

Capsules were prepared according to the formula described in Table 4.

**Table 4: Formula proportion**

| **Name of component or material** | | Formula | | | | | | | |
|---|---|---|---|---|---|---|---|---|---|
| | | Formula 37181-034-B | | Formula 37181-035-B | | Formula 37181-036-B | | Formula 37181-037-B | |
| | | Weight (mg, single capsule) | Content (wt%) | Weight (mg, single capsule) | Content (wt%) | Weight (mg, single capsule) | Content (wt%) | Weight (mg, single capsule) | Content (wt%) |
| Internal componen t | API (ATV014) | 200.00 | 55.56 | 200.00 | 55.56 | 200.00 | 55.56 | 200.00 | 55.56 |
| | Microcrystalline cellulose PH102 | 89.88 | 24.97 | 82.68 | 22.97 | 77.88 | 21.63 | 77.88 | 21.63 |
| | Mannitol 50C | 44.92 | 12.48 | 41.32 | 11.48 | 38.92 | 10.81 | 38.92 | 10.81 |
| | Croscarmellose sodium | 10.80 | 3.00 | 10.80 | 3.00 | 10.80 | 3.00 | 10.80 | 3.00 |
| | Hydroxypropyl cellulose EXF | 10.80 | 3.00 | 10.80 | 3.00 | 10.80 | 3.00 | 10.80 | 3.00 |
| | Sodium lauryl sulfate Kolliphor SLS Fine | 0 | 0 | 10.80 | 3.00 | 18.00 | 5.00 | 18.00 | 5.00 |
| External componen t | Magnesium stearate (external) | 3.60 | 1.00 | 3.60 | 1.00 | 3.60 | 1.00 | 3.60 | 1.00 |
| Total | | 360.00 | 100.0 | 360.00 | 100.0 | 360.00 | 100.0 | 360.00 | 100.0 |
| #0 gelatin hard capsule shell (white opaque) | | 1 capsule | NA | 1 capsule | NA | 1 capsule | NA | 1 capsule | NA |
| Particle size of ATV014 after grinding | | D10 = 0.775 µm; D50 = 6.160 µm; D90 = 21.332 µm | | | | | | ATV014 without grinding D10 = 77.6 µm; D50 = 257 µm; D90 = 546 µm | |

Preparation method: ATV014 was ground (not ground in formula 37181-037-B), and ATV014 and other components were sieved. The internal components were mixed according to the formula proportion. Water was added, and wet granulation was performed. The particles were ground, dried, and subjected to dry grinding. The external component was added. The components were mixed and subjected to capsule filling to give a capsule.

Dissolution detection: The obtained capsule was taken for dissolution detection using 900 mL of 0.1 N hydrochloric acid as the medium and a slurry conversion methodslurry conversion method at 75 rpm at 5 min, 15 min, 30 min, 45 min, 60 min, and 75 min, respectively. The results are shown in Table 5.

**Table 5: Results for dissolution detection**

| **Dissolution detection (slurry conversion method, medium: 0.1 N hydrochloric acid, 900 ml; N = 3)** | | | | | | | |
|---|---|---|---|---|---|---|---|
| **Formula** | **Test items** | **5 min** | **15 min** | **30 min** | **45 min** | **60 min** | **75 min** |
| Formula 37181-034-B (API ground, without SDS) | Dissolution (%) | 50 | 83 | 91 | 94 | 95 | 101 |
| | RSD% | 22.0 | 4.9 | 2.5 | 0.8 | 0.5 | 1.0 |
| Formula 37181-035-B (API ground, containing 3% SDS) | Dissolution (%) | 20 | 44 | 59 | 67 | 73 | 91 |
| | RSD% | 29.3 | 25.1 | 19.2 | 16.4 | 13.8 | 6.6 |
| Formula 37181-036-B (API ground, containing 5% SDS) | Dissolution (%) | 14 | 31 | 45 | 54 | 74 | 85 |
| | RSD% | 33.7 | 22.4 | 10.9 | 7.7 | 24.7 | 7.5 |
| Formula 37181-037-B (API not ground, containing 5% SDS) | Dissolution (%) | 11 | 24 | 35 | 41 | 46 | 77 |
| | RSD% | 4.6 | 1.4 | 1.9 | 3.6 | 4.4 | 4.2 |

Analysis of results:
(1) Formula without the addition of a surfactant (e.g., sodium lauryl sulfate (SDS)) is more beneficial for improving the dissolution rate.
(2) Grinding the API is beneficial for improving the dissolution rate.
(3) By grinding the API without the addition of a surfactant (e.g., sodium lauryl sulfate (SDS)) in synergy with each other, the dissolution rate is greatly improved.

### Example 5: Preparation Method for Jet-Milled API (Wet Granulation)

Capsules were prepared according to the formula described in Table 6.

**Table 6: Formula proportion**

| **Name of component or material** | | Formula 37181-044-B | |
|---|---|---|---|
| | | Weight (mg, single capsule) | Content (wt%) |
| Internal component | API (ATV014) | 200.00 | 55.56 |
| | Microcrystalline cellulose PH102 | 89.88 | 24.97 |
| | Mannitol 50C | 44.92 | 12.48 |
| | Croscarmellose sodium | 10.80 | 3 |
| External component | Magnesium stearate (external) | 10.80 | 3 |
| Total | | 360.00 | 100.0 |
| Empty hard gelatin capsule, #0, white opaque | | 1 capsule | |
| Particle size of ATV014 after jet milling | | D90 = 4.57-6.03 µm | |

Preparation method: ATV014 was ground, and ATV014 and other components were sieved. The internal components were mixed according to the formula proportion. Water was added, and wet granulation was performed. The particles were ground, dried, and subjected to dry grinding. The external component was added. The components were mixed and subjected to capsule filling to give a capsule.

Dissolution detection: The obtained capsule was taken for dissolution detection using 900 mL of 0.1 N hydrochloric acid as the medium and a slurry conversion method at 75 rpm at 5 min, 15 min, 30 min, 45 min, 60 min, and 75 min, respectively. The results are shown in Table 7.

**Table 7: Results for dissolution detection**

| **Dissolution detection (slurry conversion method, medium: 0.1 N hydrochloric acid, 900 ml; N = 3)** | | | | | | | |
|---|---|---|---|---|---|---|---|
| **Formula** | **Test items** | **5 min** | **15 min** | **30 min** | **45 min** | **60 min** | **75 min** |
| Formula 37181-044-B | Dissolution (%) | 60 | 94 | 97 | 98 | 100 | 100 |
| | RSD% | 15.1 | 1.6 | 2.5 | 2.0 | 1.0 | 1.2 |

Analysis of results:
1. The formula containing jet-milled API and no SDS has a fast dissolution rate and a high dissolution platform.

Example 6: Effect of ATV014 Grinding on Dissolution (Preparation Method being Dry Granulation)

Capsules were prepared according to the formula described in Table 8.

**Table 8: Formula proportion**

| **Name of component or material** | | Formula | | | |
|---|---|---|---|---|---|
| | | Formula 37181-027B | | Formula 37181-028B | |
| | | Weight (mg, single capsule) | Content (wt%) | Weight (mg, single capsule) | Content (wt%) |
| Internal component | API (ATV014) | 200.00 | 55.56 | 55.56 | 55.56 |
| | Microcrystalline cellulose PH102 | 125.80 | 34.94 | 34.94 | 34.94 |
| | Croscarmellose sodium VIVASOL | 10.80 | 3.00 | 3.00 | 3.00 |
| | Hydroxypropyl cellulose EXF | 18.00 | 5.00 | 5.00 | 5.00 |
| | Magnesium stearate (internal) | 1.80 | 0.50 | 0.50 | 0.50 |
| External component | Magnesium stearate (external) | 3.60 | 1.00 | 1.00 | 1.00 |
| Total | | 360 | 100.00 | 360 | 100.00 |
| Empty hard gelatin capsule, #4, white opaque | | 1 capsule | NA | NA | NA |
| Empty hard gelatin capsule, #0, white opaque | | NA | NA | 1 capsule | NA |
| Particle size of ATV014 after grinding | | ATV014 without grinding | | D (10): 0.811 µm | |
| | | D 10: 77.6 µm | | D (50): 5.897 µm | |
| | | D 50: 257 µm | | D (90): 19.976 µm | |
| | | D 90: 546 µm | | | |

Preparation method: ATV014 was ground (not ground in formula 37181-027B), and ATV014 and other components were sieved. The internal components were mixed according to the formula proportion. Dry granulation was performed. The external component was added. The components were mixed and subjected to capsule filling to give a capsule.

Dissolution detection: The obtained capsule was taken for dissolution detection using 900 mL of 0.1 N hydrochloric acid as the medium and a slurry conversion method at 75 rpm at 5 min, 15 min, 30 min, 45 min, 60 min, and 75 min, respectively. The results are shown in Table 9.

**Table 9: Dissolution results**

| **Dissolution results (Paddle, Medium: 0.1 N HCL, 900ml, N = 3)** | | | | | | | | |
|---|---|---|---|---|---|---|---|---|
| **Formula** | **Specification** | **Test items** | **5 min** | **15 min** | **30 min** | **45 min** | **60 min** | **75 min** |
| Formula 37181-027B | 200mg | Dissolution (%) | 42 | 63 | 74 | 81 | 86 | 94 |
| | | RSD% | 11.7 | 7.2 | 5.1 | 2.8 | 2.6 | 0.7 |
| Formula 37181-028B | 200 mg | Dissolution (%) | 64 | 87 | 91 | 93 | 93 | 101 |
| | | RSD% | 9.2 | 5.7 | 4.5 | 4.2 | 3.6 | 1.3 |

Analysis of results:
(1) Compared with wet granulation, dry granulation is more beneficial for improving the dissolution of the product compared with wet granulation.
(2) Grinding the API is beneficial for improving the dissolution of the product.

### Example 7: Effect of ATV014 Grinding on Dry Granulation Particle and Disintegration Time (Preparation Method being Dry Granulation or Direct Filling)

Capsules were prepared according to the formula described in Table 10.

**Table 10: Formula proportion**

| **Name of component or material** | | Formula | | | | | |
|---|---|---|---|---|---|---|---|
| | | Formula 37181-019-B | | Formula 37181-020-B | | Formula 37181-021-B | |
| | | Weight (mg, single capsule ) | Content (wt%) | Weight (mg, single capsule) | Content (wt%) | Weight (mg, single capsule) | Conten t (wt%) |
| Internal compon ent | API (ATV014) | 200.00 | 55.56 | 200.00 | 55.56 | 200.00 | 55.56 |
| | Microcrystalline cellulose PH102 | 125.80 | 34.94 | 125.80 | 34.94 | 125.80 | 34.94 |
| | Croscarmellose sodium VIVASOL | 10.80 | 3.00 | 10.80 | 3.00 | 10.80 | 3.00 |
| | Hydroxypropyl cellulose EXF | 18.00 | 5.00 | 18.00 | 5.00 | 18.00 | 5.00 |
| | Magnesium stearate (internal) | 1.80 | 0.50 | 1.80 | 0.50 | 1.80 | 0.50 |
| External compon ent | Magnesium stearate (external) | 3.60 | 1.00 | 3.60 | 1.00 | 3.60 | 1.00 |
| Total | | 360 | 100.00 | 360 | 100.00 | 360 | 100.00 |
| Empty hard gelatin capsule, #0, white opaque | | 1 capsule | NA | 1 capsule | NA | 1 capsule | NA |
| Particle size of ATV014 after grinding | | ATV014 without grinding | | D10: 0.891 µm | | | |
| | | | | D50: 6.267 µm | | D10: 0.891 µm | |
| | | | | | D10: 77.6 µm | D90: 22.279 µm | D50: 6.267 µm |
| | | | | | D50: 257 µm | | D90: 22.279 µm |
| | | | | | D90: 546 µm | | |

**Preparation method of formula 37181-020-B:** ATV014 was ground, and ATV014 and other components were sieved. The internal components were mixed according to the formula proportion. Dry granulation was performed. The external component was added. The components were mixed and subjected to capsule filling to give a capsule.

**Preparation method of formula 37181-019-B:** ATV014 which was not ground and other components were separately sieved. The internal components were mixed according to the formula proportion. The external component was then added. The components were mixed and subjected to capsule filling to give a capsule.

**Preparation method of formula 37181-021-B:** ATV014 was ground, and ATV014 and other components were sieved. The internal components were mixed according to the formula proportion. The external component was then added. The components were mixed and subjected to capsule filling to give a capsule.

**Observation of dry granulation:** The dry granulation process parameters shown in Table 11 were used for granulation, and the properties of the material were observed during granulation. The results are shown in Table 11.

**Table 11: Results for observation of dry granulation**

| **Observation of dry granulation** | | | |
|---|---|---|---|
| **Formula** | **Parameter** | | **Observation** |
| | **Roll pressure (kg/cm²)** | **Roll speed (rpm)** | |
| Formula 37181-019-B (ATV014 not ground) | 30-40 | 0.96 | No adhesion, less particles, and relatively soft particle hardness |
| | 40-50 | 0.96 | No adhesion, less particles, and relatively soft particle hardness |
| | 50-60 | 0.96 | No adhesion, less particles, and relatively soft particle hardness |
| | 60-70 | 0.96 | No adhesion, less particles, and relatively soft particle hardness |
| Formula 37181-020-B (ATV014 ground) | 20-30 | 0.96 | No adhesion and moderate particle hardness |
| | 30-40 | 0.96 | No adhesion and hard particle hardness |

Brief summary: Using the ground ATV014 for dry granulation is beneficial for giving particles with relatively good hardness.

**Investigation of disintegration time:** The disintegration time of the capsules obtained in this example was measured according to General Chapter 0921, *Chinese Pharmacopoeia,* Volume IV, 2020 Edition. The results are shown in Table 12.

**Table 12: Results for investigation of disintegration time**

| **Capsule content weight and disintegration time** | | | | | |
|---|---|---|---|---|---|
| **Formula** | **Capsule shell** | **SpecificationSpecification (mg)** | **Prefilling amount (mg) before pressure filling** | **Actual filling amount (filling capsule body with pressure) (mg)** | **Disintegration time** |
| 37181-019-B (200 mg, API not ground, direct filling) | #0 | 360 | 311.9 | 445.5 | 2 min and 10 s |
| 37181-020-B (200 mg, API ground, filling after dry granulation) | #0 | 360 | 292.3 | 462.7 | 3 min and 02 s |
| 37181-021-B (200 mg, API ground, direct filling) | #0 | 360 | 165.5 | 412.3 | 6 min and 19 s |

| | | | | | |
|---|---|---|---|---|---|
| Conclusion: The disintegration time of the capsule prepared by the preparation method of grinding API and filling after dry granulation is fast, and dry granulation is beneficial for improving the disintegration rate of the product. | | | | | |

### Example 8: Investigation of Binder and Lubricant Content

Capsules were prepared according to the formula described in Table 13.

**Table 13: Formula proportion**

| **Name of component or material** | | Formula | | | | | |
|---|---|---|---|---|---|---|---|
| | | Formula 37181-047-B | | Formula 37181-050-B | | Formula 37181-051-B | |
| | | Weight (mg, single capsule) | Content (wt%) | Weight (mg, single capsule) | Weight/weight (wt%) | Weight (mg, single capsule) | Content (wt%) |
| Internal component | API (ATV014) | 200.00 | 55.56 | 200.00 | 55.56 | 200.00 | 55.56 |
| | Microcrystalline cellulose PH102 | 125.8 | 34.94 | 124.00 | 34.44 | 131.20 | 36.44 |
| | Croscarmellose sodium VIVASOL | 10.8 | 3.00 | 10.80 | 3.00 | 10.80 | 3.00 |
| | Hydroxypropyl cellulose EXF | 18 | 5.00 | 18.00 | 5.00 | 10.8 | 3.00 |
| | Magnesium stearate (internal) | 3.6 | 1.00 | 5.40 | 1.50 | 5.4 | 1.50 |
| External component | Magnesium stearate (external) | 1.8 | 0.50 | 1.80 | 0.5 | 1.8 | 0.5 |
| Total | | 360 | 100.00 | 360.00 | 100.00 | 360.00 | 100.00 |
| Empty hard gelatin capsule, #4 white opaque | | NA | NA | NA | NA | NA | NA |
| Empty hard gelatin capsule, #0 white opaque | | 1 capsule | NA | NA | 1 capsule | NA | NA |
| Particle size of API (ATV014) after grinding | | D 90: 4.57-6.03 µm | | D 10: 1.03 µm | | D 10: 1.03 µm | |
| | | | | D 50: 2.76 µm | | D 50: 2.76 µm | |
| | | | | D 90: 7.30 µm | | D 90: 7.30 µm | |

Preparation method: ATV014 was ground, and ATV014 and other components were sieved. The internal components were mixed according to the formula proportion. Dry granulation was performed. The external component was added. The components were mixed and subjected to capsule filling to give a capsule.

Observation of dry granulation: The dry granulation process parameters shown in Table 14 were used for granulation, and the properties of the material were observed during granulation. The results are shown in Table 14.

**Table 14: Observation of dry granulation**

| **Observation of dry granulation** | | | |
|---|---|---|---|
| **Formula** | **Parameter** | | **Observation** |
| | Roll pressure (kg/cm²) | Roll speed (rpm) | |
| Formula 37181-047-B (containing 5% hydroxypropyl cellulose + 1% magnesium stearate (internal)) | 20-30 | 0.96 | No adhesion and relatively hard particle hardness |
| | 10-20 | 0.96 | No adhesion, with cracking, and relatively hard particle hardness |
| | 6-12 | 0.96 | A little adhesion and relatively hard particle hardness |
| Formula 37181-050-B (containing 5% hydroxypropyl cellulose + 1.5% magnesium stearate (internal)) | 6-12 | 0.96 | Moderate particle hardness, very round, and smooth |
| Formula 37181-051-B (containing 3% hydroxypropyl cellulose + 1% magnesium stearate (internal)) | 6-12 | 0.96 | Moderate particle hardness but softer than that of formula 37181-050-B, very round, and smooth |

### Brief summary:

1. The content of magnesium stearate is preferably 1.5 wt% or more, which is beneficial for the hardness and smoothness of the dry granulated particles.
2. The content of hydroxypropyl cellulose is preferably 3 wt% or more, which is beneficial for the hardness and smoothness of the dry granulated particles.

Dissolution detection: The capsule obtained with the roll pressure of 6-12 kg/cm ² was taken for dissolution detection using 900 mL of 0.1 N hydrochloric acid as the medium and a slurry conversion method at 75 rpm at 5 min, 15 min, 30 min, 45 min, 60 min, and 75 min, respectively. The results are shown in Table 15.

**Table 15: Dissolution results**

| **Dissolution detection (slurry conversion method, medium: 0.1 N hydrochloric acid, 900 ml; N = 3)** | | | | | | | |
|---|---|---|---|---|---|---|---|
| **Formula** | **Test items** | **5 min** | **15 min** | **30 min** | **45 min** | **60 min** | **75 min** |
| Formula 37181-047-B | Dissolution (%) | 55 | 86 | 93 | 95 | 96 | 99 |
| | RSD% | 15.4 | 3.6 | 2.6 | 2.0 | 1.4 | 1.6 |
| Formula 37181-051-B | Dissolution (%) | 64 | 92 | 96 | 96 | 99 | 99 |
| | RSD% | 2.6 | 1.8 | 1.3 | 1.5 | 0.9 | 0.4 |

| | | | | | | | |
|---|---|---|---|---|---|---|---|
| Conclusion: The dissolution of both the capsules obtained by formula 37181-047-B and formula 37181-051-B can be quick and complete, wherein the releasing rate of formula 37181-051-B is faster. | | | | | | | |

### Example 9: Capsules with Different Specifications

### Capsules were prepared according to the formula described in Table 16.

**Table 16: Formula proportion**

| **Name of component or material** | | Formula | | | |
|---|---|---|---|---|---|
| | | Formula 37181-054A | | Formula 37181-054B | |
| | | Weight (mg, single capsule) | Content (wt%) | Weight (mg, single capsule) | Content (wt%) |
| Internal component | API (ATV014) | 50.00 | 55.56 | 200.00 | 55.56 |
| | Microcrystalline cellulose PH102 | 32.8 | 36.44 | 131.2 | 36.44 |
| | Croscarmellose sodium VIVASOL | 2.70 | 3.00 | 10.80 | 3.00 |
| | Hydroxypropyl cellulose EXF | 2.70 | 3.00 | 10.80 | 3.00 |
| | Magnesium stearate (internal) | 1.35 | 1.50 | 5.4 | 1.50 |
| External component | Magnesium stearate (external) | 0.45 | 0.50 | 1.80 | 0.50 |
| Total | | 90.00 | 100.00 | 360 | 100.00 |
| Empty hard gelatin capsule, #4, white opaque | | 1 capsule | NA | NA | NA |
| Empty hard gelatin capsule, #0, white opaque | | NA | NA | 1 capsule | NA |
| Particle size of ATV014 after grinding | | D10: 1.03 µm; D50: 2.76 µm; D 90: 7.30 µm | | | |

Preparation method: ATV014 was ground, and ATV014 and other components were sieved. The internal components were mixed according to the formula proportion. Dry granulation was performed. The external component was added. The components were mixed and subjected to capsule filling to give a capsule.

Dissolution detection: The obtained capsule was taken for dissolution detection using 900 mL of 0.1 N hydrochloric acid as the medium and a slurry conversion methodslurry conversion method at 75 rpm at 5 min, 15 min, 30 min, 45 min, 60 min, and 75 min, respectively. The results are shown in Table 17.

**Table 17: Dissolution results**

| **Dissolution results (Paddle, Medium: 0.1 N HCL, 900ml, N = 3)** | | | | | | | | |
|---|---|---|---|---|---|---|---|---|
| **Formula** | **Specification** | **Test items** | **5 min** | **15 min** | **30 min** | **45 min** | **60 min** | **75 min** |
| Formula 37181-0564-A | 50 mg | Dissolution (%) | 73 | 98 | 101 | 101 | 102 | 101 |
| | | RSD% | 5.2 | 1.3 | 1.0 | 1.6 | 1.5 | 1.4 |
| Formula 37181-054-B | 200 mg | Dissolution (%) | 59 | 90 | 94 | 96 | 96 | 101 |
| | | RSD% | 12.8 | 1.0 | 1.0 | 2.2 | 2.4 | 0.8 |

| | | | | | | | | |
|---|---|---|---|---|---|---|---|---|
| Conclusion: Capsules with different specifications all have good dissolution, wherein the dissolution rate of the capsule with 50 mg specification is faster. | | | | | | | | |

### Example 10: Stability Investigation

The capsules provided by the present invention were taken and separately put under the conditions shown in Table 18 to investigate the stability. The results show that the related substances of the capsules provided by the present invention have good stability.

**Table 18: Investigation results of stability of related substance**

| **Results of related substance study** | | | | | | | | | |
|---|---|---|---|---|---|---|---|---|---|
| Item | Condition | **Impurity (%)** | | | | | | | |
| | | **Relative retention time (RRT)** | | | | | | | |
| | | **0.24** | **0.42** | **0.49 (0.48)** | **1.16** | **1.42 (1.38)** | **1.44 (1.40)** | **1.70 (1.65)** | **Total** |
| **API (ATV014)** | 0 day | N.D. | 0.47 | 0.19 | 0.75 | 0.53 | 0.16 | 0.04 | 2.10 |
| **37181-054 -A (50 mg)** | 0 day | N.D. | 0.46 | 0.2 | 0.73 | 0.53 | 0.17 | 0.04 | 2.09 |
| | 40 °C/75% RH (open) - 10 days | N.D. | 0.47 | 0.19 | 0.71 | 0.54 | 0.17 | 0.04 | 2.08 |
| | 40 °C/75% RH (sealed) - 10 days | 0.04 | 0.47 | 0.2 | 0.72 | 0.53 | 0.18 | 0.04 | 2.10 |
| | 30 °C/65% RH (sealed) - 10 days | N.D. | 0.47 | 0.2 | 0.74 | 0.53 | 0.18 | 0.04 | 2.12 |
| | Illumination (4500 lx ± 500 lx) - 10 days | N.D. | 0.47 | 0.19 | 0.73 | 0.54 | 0.17 | 0.04 | 2.10 |
| **37181-054 -B (200 mg)** | 0 day | N.D. | 0.48 | 0.19 | 0.71 | 0.54 | 0.17 | 0.04 | 2.09 |
| | 40 °C/75% RH (open) - 10 days | N.D. | 0.48 | 0.18 | 0.71 | 0.54 | 0.17 | 0.04 | 2.08 |
| | 40 °C/75% RH (sealed) - 10 days | N.D. | 0.47 | 0.19 | 0.72 | 0.54 | 0.17 | 0.04 | 2.09 |
| | 30 °C/65% RH (sealed) - 10 days | N.D. | 0.48 | 0.19 | 0.74 | 0.54 | 0.17 | 0.04 | 2.12 |
| | Illumination (4500 lx ± 500 lx) - 10 days | N.D. | 0.47 | 0.19 | 0.72 | 0.53 | 0.17 | 0.04 | 2.08 |

### Example 11: Investigation of Compatibility of Raw Material and Auxiliary Material

### Excipient compatibility study

The raw material and the auxiliary material were mixed in the proportions shown in Table 19. The mixture was allowed to stand at 60 °C high temperature condition (60 °C), high humidity condition (25 °C, 92.5% RH), acceleration condition (40 °C ± 2 °C, 75% RH ± 5%), and light condition (4500 lx ± 500 lx) for 10 days and 30 days, and then taken out to detect the content and the change of the related substance. The results are shown in Table 20.

**Table 19: Test of compatibility of raw material and auxiliary material**

| **No.** | **API/excipient** | **Mass ratio (Ratio)** |
|---|---|---|
| **1** | ATV014 | / |
| **2** | ATV014:microcrystalline cellulose PH102 | 1:5 |
| **3** | ATV014:mannitol 100 SD | 1:5 |
| **4** | ATV014:mannitol 50C | 1:5 |
| **5** | ATV014:calcium phosphate, Dibasic Anhydrous | 1:5 |
| **6** | ATV014:pregelatinized starch Starch 1500 | 1:5 |
| **7** | ATV014:croscarmellose sodium VIVASOL^{®} | 1:1 |
| **8** | ATV014:crospovidone XL-10 | 1:1 |
| **9** | ATV014:hydroxypropyl cellulose-EXF | 1:1 |
| **10** | ATV014:povidone K30 | 1:1 |
| **11** | ATV014:sodium lauryl sulfate | 5:1 |
| **12** | ATV014:magnesium stearate LIGAMED MF-2-V | 5:1 |
| **13** | ATV014:sodium stearyl fumarate PRUV | 5:1 |
| **14** | ATV014:silicon dioxide 200 Pharma | 5:1 |
| **15** | ATV014:empty capsule, 0# rich yellow opaque | 1:1 |
| **16** | ATV014:0padry^{®} II complete film coating system 85F620077-CN, US | 1:1 |

**Table 20: Content measurement results (numerical value of result in %)**

| No | **API:excipient** | **0 day** | **60 °C, open** | | **25 °C/92.5% RH, open** | | **Illuminat ion, open** | **40 °C/75% RH, open** | |
|---|---|---|---|---|---|---|---|---|---|
| | | | 10 day | 30 day | 10 day | 30 day | 10 day | 10 day | 30 day |
| 1 | ATV014 | 101. 8 | 100.3 | 100.5 | 100.3 | 99.8 | 100.9 | 102. 2 | 101. 0 |
| 2 | ATV014:microcry stalline cellulose PH102 | 102. 9 | 112.4 | 116.6 | 112.7 | 103.3 | 100.1 | 100. 8 | 100. 9 |
| 3 | ATV014:mannitol 100 SD | 96.3 | 110.3 | 108.4 | 114.3 | 104.6 | 101.4 | 94.7 | 103. 6 |
| 4 | ATV014:mannitol 50C | 101. 9 | 105.5 | 105.5 | 109.0 | 106.8 | 94.3 | 96.5 | 106. 8 |
| 5 | ATV014:calcium | 90.7 | 103.5 | N/A | 116.1 | N/A | 97.5 | 120. | N/A |
| | phosphate, Dibasic Anhydrous | | | | | | | 2 | |
| 6 | ATV014:pregelati nized starch Starch 1500 | 90.4 | 95.0 | N/A | 99.8 | N/A | 97.6 | 83.1 | N/A |
| 7 | ATV014:croscarm ellose sodium VIVASOL^{®} | 105. 3 | 106.2 | 105.0 | 105.8 | 105.3 | 105.2 | 106. 0 | 103. 2 |
| 8 | ATV014:crospovid one XL-10 | 106. 3 | 101.5 | 105.0 | 106.5 | 106.2 | 104.4 | 103. 2 | 105. 4 |
| 9 | ATV014:hydroxyp ropyl cellulose-EXF | 100. 8 | 103.4 | 99.7 | 100.8 | 98.6 | 102.4 | 97.3 | 99.1 |
| 10 | ATV014:povidone K30 | 103. 8 | 99.7 | 104.0 | 101.6 | 102.8 | 103.6 | 102. 0 | 101. 7 |
| 11 | ATV014:sodium lauryl sulfate | 103. 8 | 102.2 | 102.6 | 104.2 | 103.7 | 106.8 | 102. 4 | 103. 9 |
| 12 | ATV014:magnesiu m stearate LIGAMED MF-2-V | 100. 5 | 101.6 | 99.1 | 100.1 | 99.0 | 99.8 | 100. 9 | 99.4 |
| 13 | ATV014:sodium stearyl fumarate PRUV | 102. 4 | 104.3 | 100.0 | 102.8 | 104.1 | 102.9 | 102. 9 | 104. 3 |
| 14 | ATV014:silicon dioxide 200 Pharma | 104. 4 | 102.6 | 104.5 | 103.9 | 106.8 | 103.9 | 104. 3 | 108. 4 |
| 15 | ATV014:empty capsule, 0# rich yellow opaque | 97.7 | 96.5 | 96.8 | 97.0 | 95.5 | 97.3 | 96.3 | 95.7 |
| 16 | ATV014:Opadry^{®} II complete film coating system 85F620077-CN, US | 99.9 | 100.2 | N/A | 99.6 | N/A | 101.0 | 102. 6 | N/A |

N/A: According to the EC scheme, it is an alternative auxiliary material and is not detected.

### Conclusion:

Detection of related substance: The selected auxiliary material in the present invention and ATV014 were allowed to stand for 30 days under the condition of acceleration (40 °C/75% ± 5% RH), high humidity (25 °C/90% ± 5% RH), or high temperature (60 °C) and allowed to stand for 10 days under the condition of illumination (visible light not less than 1.2 × 106 Lux·hr, near ultraviolet not less than 200 w·hr/m2) without obvious change of impurities, and all the auxiliary materials and the raw material had relatively good compatibility.

From the results in Table 20, it can be seen that:

In the investigation of the diluent (Nos. 2-6), microcrystalline cellulose PH102 was used, which is beneficial for the stability of ATV014 under the conditions of high temperature (60 °C) and illumination (visible light not less than 1.2 × 106 Lux·hr, near ultraviolet not less than 200 w·hr/m2). Compared with other diluents, mannitol 50C was more beneficial for the stability of ATV014 under the 60 °C high temperature condition (60°C), the high humidity condition (25 °C, 92.5% RH), the acceleration condition (40 ± 2 °C, 75% RH ± 5%), and the illumination condition (4500 lx ± 500 lx), and had unexpected technical effects.

In the investigation of the disintegrant (Nos. 7-8), using croscarmellose sodium VIVASOL^{®} or crospovidone XL-10 was both beneficial for the stability of ATV014 under the 60 °C high temperature condition (60°C), the high humidity condition (25 °C, 92.5% RH), the acceleration condition (40 ± 2 °C, 75% RH ± 5%), and the illumination condition (4500 lx ± 500 lx).

In the investigation of the binder (Nos. 9-10), using hydroxypropyl cellulose-EXF or povidone K30 was both beneficial for the stability of ATV014 under the 60 °C high temperature condition (60 °C), the high humidity condition (25 °C, 92.5% RH), the acceleration condition (40 ± 2 °C, 75% RH ± 5%), and the illumination condition (4500 lx ± 500 lx).

In the investigation of the surfactant (No. 11), using sodium lauryl sulfate was beneficial for the stability under the 60 °C high temperature condition (60 °C), the high humidity condition (25 °C, 92.5% RH), the acceleration condition (40 ± 2 °C, 75% RH ± 5%), and the illumination condition (4500 lx ± 500 lx).

In the investigation of the lubricant (Nos. 12-13), using magnesium stearate LIGAMED MF-2-V or sodium stearyl fumarate PRUV was both beneficial for the stability of ATV014 under the 60 °C high temperature condition (60 °C), the high humidity condition (25 °C, 92.5% RH), the acceleration condition (40 ± 2 °C, 75% RH ± 5%), and the illumination condition (4500 lx ± 500 lx).

In the investigation of the glidant (No. 14), using silicon dioxide 200 Pharma was beneficial for the stability of ATV014 under the 60 °C high temperature condition (60 °C), the high humidity condition (25 °C, 92.5% RH), the acceleration condition (40 ± 2 °C, 75% RH ± 5%), and the illumination condition (4500 lx ± 500 lx).

In the investigation of the capsule material and the coating material (Nos. 15-16), using empty capsule, 0# rich yellow opaque or Opadry^{®} II complete film coating system 85F620077-CN, US was beneficial for the stability of ATV014 under the 60 °C high temperature condition (60 °C), the high humidity condition (25 °C, 92.5% RH), the acceleration condition (40 ± 2 °C, 75% RH ± 5%), and the illumination condition (4500 lx ± 500 lx).

## Claims

1. A composition, comprising: a compound ATV014 and a pharmaceutically acceptable excipient, 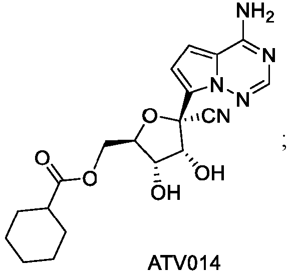 wherein the pharmaceutically acceptable excipients comprise at least one selected from a diluent, a disintegrant, a binder and a lubricant, and wherein the composition does not comprise a surfactant.

2. The composition according to claim 1, wherein the pharmaceutically acceptable excipient comprises other auxiliary materials.

3. The composition according to claim 2, wherein the diluent comprises at least one selected from microcrystalline cellulose, mannitol, and pregelatinized starch; preferably, the dilution is microcrystalline cellulose or mannitol; more preferably, the dilution is microcrystalline cellulose PH102 or mannitol 50C; most preferably, the diluent is mannitol 50C; and/or
the disintegrant comprises at least one selected from croscarmellose sodium, crospovidone, sodium starch glycolate, and hydroxypropyl cellulose; and/or
the binder comprises at least one selected from hydroxypropyl cellulose, povidone, and starch; and/or
the lubricant comprises at least one selected from magnesium stearate, stearic acid, and sodium stearyl fumarate; and/or
the other auxiliary materials comprise an auxiliary material selected from a glidant; and/or
the glidant comprises a glidant selected from colloidal silicon dioxide or talc; and/or
the composition further comprises an external lubricant; and/or
the external lubricant comprises at least one selected from magnesium stearate, sodium stearate, and sodium stearyl fumarate.

4. The composition according to any one of claims 2-3, wherein the content of the compound ATV014 is 15 wt% to 70 wt%, based on the total mass of the composition; and/or
the content of the diluent is 20 wt% to 70 wt%, based on the total mass of the composition; and/or
the content of the disintegrant is 1 wt% to 10 wt%, based on the total mass of the composition; and/or
the content of the binder is 1 wt% to 10 wt%, based on the total mass of the composition; and/or
the content of the lubricant is 0.5 wt% to 5 wt%, based on the total mass of the composition; and/or
the content of the external lubricant is 0.5 wt% to 5 wt%, based on the total mass of the composition; and/or
the composition does not comprise sodium lauryl sulfate.

5. The composition according to any one of claims 2-3, comprising: a compound ATV014 and a pharmaceutically acceptable excipient, the pharmaceutically acceptable excipient comprising at least one selected from a diluent, a disintegrant, a binder, a lubricant, and other auxiliary materials, the diluent comprising at least one selected from microcrystalline cellulose, mannitol, calcium hydrophosphate, and pregelatinized starch, the disintegrant comprising at least one selected from croscarmellose sodium, crospovidone, sodium starch glycolate, and hydroxypropyl cellulose, the binder comprising at least one selected from hydroxypropyl cellulose, povidone, and starch, and the lubricant comprising at least one selected from magnesium stearate, stearic acid, and sodium stearyl fumarate, wherein based on the total mass of the composition, the content of the compound ATV014 is 15 wt% to 70 wt%, the content of the diluent is 20 wt% to 70 wt%, the content of the disintegrant is 1 wt% to 10 wt%, the content of the binder is 1 wt% to 10 wt%, and the content of the lubricant is 0.5 wt% to 5 wt%; or
comprising: a compound ATV014 and a pharmaceutically acceptable excipient, the pharmaceutically acceptable excipient comprising at least one selected from a diluent, a disintegrant, a binder, a lubricant, and other auxiliary materials, the diluent comprising at least one selected from microcrystalline cellulose, mannitol, calcium hydrophosphate, and pregelatinized starch, the disintegrant comprising at least one selected from croscarmellose sodium, crospovidone, sodium starch glycolate, and hydroxypropyl cellulose, the binder comprising at least one selected from hydroxypropyl cellulose, povidone, and starch, and the lubricant comprising at least one selected from magnesium stearate, stearic acid, and sodium stearyl fumarate, wherein based on the total mass of the composition, the content of the compound ATV014 is 50 wt% to 60 wt%, the content of the diluent is 30 wt% to 40 wt%, the content of the disintegrant is 2 wt% to 5 wt%, the content of the binder is 2 wt% to 5 wt%, and the content of the lubricant is 1.5% to 5 wt%; or
comprising: a compound ATV014 and a pharmaceutically acceptable excipient, the pharmaceutically acceptable excipient comprising at least one selected from a diluent, a disintegrant, a binder, a lubricant, and other auxiliary materials, the diluent comprising at least one selected from microcrystalline cellulose and mannitol, the disintegrant comprising a disintegrant selected from croscarmellose sodium, the binder comprising a binder selected from hydroxypropyl cellulose, and the lubricant comprising at least one selected from magnesium stearate, stearic acid, and sodium stearyl fumarate, wherein based on the total mass of the composition, the content of the compound ATV014 is 50 wt% to 60 wt%, the content of the diluent is 30 wt% to 40 wt%, the content of the disintegrant is 2 wt% to 5 wt%, the content of the binder is 2 wt% to 5 wt%, and the content of the lubricant is 1.5% to 5 wt%; or
comprising: a compound ATV014 and a pharmaceutically acceptable excipient, the pharmaceutically acceptable excipient comprising at least one selected from a diluent, a disintegrant, a binder, a lubricant, and other auxiliary materials, the diluent being microcrystalline cellulose PH102 or mannitol 50C, the disintegrant comprising a disintegrant selected from croscarmellose sodium, the binder comprising a binder selected from hydroxypropyl cellulose, and the lubricant comprising at least one selected from magnesium stearate, stearic acid, and sodium stearyl fumarate, wherein based on the total mass of the composition, the content of the compound ATV014 is 50 wt% to 60 wt%, the content of the diluent is 30 wt% to 40 wt%, the content of the disintegrant is 2 wt% to 5 wt%, the content of the binder is 2 wt% to 5 wt%, and the content of the lubricant can be 1.5% to 5 wt%; or
comprising: a compound ATV014 and a pharmaceutically acceptable excipient, the pharmaceutically acceptable excipient comprising at least one selected from a diluent, a disintegrant, a binder, a lubricant, and other auxiliary materials, the diluent being microcrystalline cellulose PH102 and mannitol 50C, the disintegrant comprising a disintegrant selected from croscarmellose sodium, the binder comprising a binder selected from hydroxypropyl cellulose, and the lubricant comprising at least one selected from magnesium stearate, stearic acid, and sodium stearyl fumarate, wherein based on the total mass of the composition, the content of the compound ATV014 is 50 wt% to 60 wt%, the content of the diluent is 30 wt% to 40 wt%, the content of the disintegrant is 2 wt% to 5 wt%, the content of the binder is 2 wt% to 5 wt%, and the content of the lubricant is 1.5% to 5 wt%; or
comprising: a compound ATV014 and a pharmaceutically acceptable excipient, the pharmaceutically acceptable excipient comprising at least one selected from a diluent, a disintegrant, a binder, a lubricant, and other auxiliary materials, the diluent being mannitol 50C, the disintegrant comprising a disintegrant selected from croscarmellose sodium, the binder comprising a binder selected from hydroxypropyl cellulose, and the lubricant comprising at least one selected from magnesium stearate, stearic acid, and sodium stearyl fumarate, wherein based on the total mass of the composition, the content of the compound ATV014 is 50 wt% to 60 wt%, the content of the diluent is 30 wt% to 40 wt%, the content of the disintegrant is 2 wt% to 5 wt%, the content of the binder is 2 wt% to 5 wt%, and the content of the lubricant is 1.5% to 5 wt%.

6. The composition according to any one of claims 1-3, wherein the dosage form of the composition is a solid oral dosage form or an injection;
optionally, the dosage form is a tablet, a granule, or a capsule.

7. Use of the composition according to any one of claims 1-6 in the preparation of a product for preventing, alleviating or treating a coronavirus infection, or replication or propagation of a homologous variant virus thereof, and a cytopathic effect generated therefrom, or a product for detecting a coronavirus or a homologous variant virus thereof.

8. The use according to claim 7, wherein the infection comprises fever, cough, sore throat, pneumonia, acute respiratory infection, severe acute respiratory infection, hypoxic respiratory failure and acute respiratory distress syndrome, sepsis, or septic shock.

9. The use according to any one of claims 7-8, wherein the coronavirus comprises: MHV-A59, HCoV-229E, HCoV-OC43, HCoV-NL63, HCoV-HKU1, SARS-CoV, MERS-CoV, SARS-CoV-2, a mouse hepatitis virus, a feline infectious peritonitis virus, a canine coronavirus, a bovine coronavirus, an avian infectious bronchitis virus, or a porcine coronavirus; preferably, the SARS-CoV-2 comprises a mutant strain or a non-mutant strain of SARS-CoV-2; more preferably, the mutant strain of SARS-CoV-2 comprises SARS-CoV-2 mutant strain B.1, SARS-CoV-2 mutant strain B.1.351, SARS-CoV-2 mutant strain B.1.617.2, SARS-CoV-2 mutant strain C.37, SARS-CoV-2 mutant strain P.1 lineage, SARS-CoV-2 mutant strain B.1.525, SARS-CoV-2 mutant strain B.1.427, or SARS-CoV-2 mutant strain B.1.429.

10. The use according to any one of claims 7-8, wherein the composition is suitable for use in humans or animals; and/or
the animal comprises bovine, equine, ovine, porcine, canine, feline, rodent, primate, avian, or piscine animal.

11. A method for preparing the composition according to any one of claims 1-6, comprising: mixing the compound ATV014 and the pharmaceutically acceptable excipient, performing dry granulation, adding an external lubricant, mixing, and tableting or capsule filling or packaging to give the composition; or
mixing the compound ATV014 and the pharmaceutically acceptable excipient, performing wet granulation, grinding, drying, dry grinding, adding an external lubricant, mixing, and tableting or capsule filling or packaging to give the composition; or
grinding or pulverizing the compound ATV014, mixing the compound and the pharmaceutically acceptable excipient, performing dry granulation, adding an external lubricant, mixing, and tableting or capsule filling or packaging to give the composition; or
grinding or pulverizing the compound ATV014, mixing the compound and the pharmaceutically acceptable excipient, performing wet granulation, grinding, drying, dry grinding, adding an external lubricant, mixing, and tableting or capsule filling or packaging to give the composition.

## Patentansprüche

1. Zusammensetzung, umfassend: eine Verbindung ATV014 und einen pharmazeutisch annehmbaren Hilfsstoff, 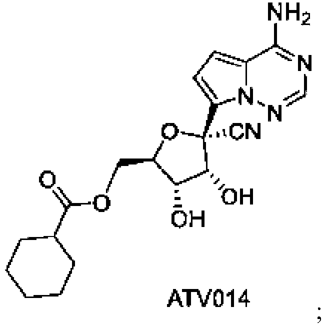 wobei die pharmazeutisch annehmbaren Hilfsstoffe mindestens einen ausgewählt aus einem Verdünnungsmittel, einem Zerfallsmittel, einem Bindemittel und einem Schmiermittel umfassen und wobei die Zusammensetzung kein Tensid umfasst.

2. Zusammensetzung nach Anspruch 1, wobei der pharmazeutisch annehmbare Hilfsstoff weitere Zusatzmaterialien umfasst.

3. Zusammensetzung nach Anspruch 2, wobei das Verdünnungsmittel mindestens eines ausgewählt aus mikrokristalliner Cellulose, Mannitol und vorverkleisterter Stärke umfasst; vorzugsweise ist das Verdünnungsmittel mikrokristalline Cellulose oder Mannitol; besonders bevorzugt ist das Verdünnungsmittel mikrokristalline Cellulose PH102 oder Mannitol 50C; am bevorzugtesten ist das Verdünnungsmittel Mannitol 50C; und/oder das Zerfallsmittel mindestens eines ausgewählt aus Croscarmellose-Natrium, Crospovidon, Natriumstärkeglycolat und Hydroxypropylcellulose umfasst; und/oder
das Bindemittel mindestens eines ausgewählt aus Hydroxypropylcellulose, Povidon und Stärke umfasst; und/oder
das Schmiermittel mindestens eines ausgewählt aus Magnesiumstearat, Stearinsäure und Natriumstearylfumarat umfasst; und/oder die weiteren Zusatzmaterialien ein aus einem Gleitmittel ausgewähltes Zusatzmaterial umfassen; und/oder
das Gleitmittel ein aus kolloidalem Siliciumdioxid oder Talk ausgewähltes Gleitmittel umfasst; und/oder
wobei die Zusammensetzung ferner ein externes Schmiermittel umfasst; und/oder
das externe Schmiermittel mindestens eines ausgewählt aus Magnesiumstearat, Natriumstearat und Natriumstearylfumarat umfasst.

4. Zusammensetzung nach einem der Ansprüche 2 bis 3, wobei der Gehalt der Verbindung ATV014 15 Gew.-% bis 70 Gew.-% beträgt, bezogen auf die Gesamtmasse der Zusammensetzung; und/oder
der Gehalt des Verdünnungsmittels 20 Gew.-% bis 70 Gew.-% beträgt, bezogen auf die Gesamtmasse der Zusammensetzung; und/oder
der Gehalt des Zerfallsmittels 1 Gew.-% bis 10 Gew.-% beträgt, bezogen auf die Gesamtmasse der Zusammensetzung; und/oder
der Gehalt des Bindemittels 1 Gew.-% bis 10 Gew.-% beträgt, bezogen auf die Gesamtmasse der Zusammensetzung; und/oder
der Gehalt des Schmiermittels 0,5 Gew.-% bis 5 Gew.-% beträgt, bezogen auf die Gesamtmasse der Zusammensetzung; und/oder
der Gehalt des externen Schmiermittels 0,5 Gew.-% bis 5 Gew.-% beträgt, bezogen auf die Gesamtmasse der Zusammensetzung; und/oder wobei die Zusammensetzung kein Natriumlaurylsulfat umfasst.

5. Zusammensetzung nach einem der Ansprüche 2 bis 3, umfassend: eine Verbindung ATV014 und einen pharmazeutisch annehmbaren Hilfsstoff, wobei der pharmazeutisch annehmbare Hilfsstoff mindestens einen ausgewählt aus einem Verdünnungsmittel, einem Zerfallsmittel, einem Bindemittel, einem Schmiermittel und weiteren Zusatzmaterialien umfasst, wobei das Verdünnungsmittel mindestens eines ausgewählt aus mikrokristalliner Cellulose, Mannitol, Calciumhydrophosphat und vorverkleisterter Stärke umfasst, wobei das Zerfallsmittel mindestens eines ausgewählt aus Croscarmellose-Natrium, Crospovidon, Natriumstärkeglycolat und Hydroxypropylcellulose umfasst, das Bindemittel mindestens eines ausgewählt aus Hydroxypropylcellulose, Povidon und Stärke umfasst und das Schmiermittel mindestens eines ausgewählt aus Magnesiumstearat, Stearinsäure und Natriumstearylfumarat umfasst, wobei jeweils der Gehalt der Verbindung ATV014 15 Gew.-% bis 70 Gew.-% beträgt, der Gehalt des Verdünnungsmittels 20 Gew.-% bis 70 Gew.-% beträgt, der Gehalt des Zerfallsmittels 1 Gew.-% bis 10 Gew.-% beträgt, der Gehalt des Bindemittels 1 Gew.-% bis 10 Gew.-% beträgt und der Gehalt des Schmiermittels 0,5 Gew.-% bis 5 Gew.-% beträgt; bezogen auf die Gesamtmasse der Zusammensetzung, oder
wobei die Zusammensetzung umfasst: eine Verbindung ATV014 und einen pharmazeutisch annehmbaren Hilfsstoff, wobei der pharmazeutisch annehmbare Hilfsstoff mindestens einen ausgewählt aus einem Verdünnungsmittel, einem Zerfallsmittel, einem Bindemittel, einem Schmiermittel und weiteren Zusatzmaterialien umfasst, wobei das Verdünnungsmittel mindestens eines ausgewählt aus mikrokristalliner Cellulose, Mannitol, Calciumhydrophosphat und vorverkleisterter Stärke umfasst, wobei das Zerfallsmittel mindestens eines ausgewählt aus Croscarmellose-Natrium, Crospovidon, Natriumstärkeglycolat und Hydroxypropylcellulose, umfasst, das Bindemittel mindestens eines ausgewählt aus Hydroxypropylcellulose, Povidon und Stärke umfasst und das Schmiermittel mindestens eines ausgewählt aus Magnesiumstearat, Stearinsäure und Natriumstearylfumarat umfasst, wobei, jeweils der Gehalt der Verbindung ATV014 50 Gew.-% bis 60 Gew.-% beträgt, der Gehalt des Verdünnungsmittels 30 Gew.-% bis 40 Gew.-% beträgt, der Gehalt des Zerfallsmittels 2 Gew.-% bis 5 Gew.-% beträgt, der Gehalt des Bindemittels 2 Gew.-% bis 5 Gew.-% beträgt und der Gehalt des Schmiermittels 1,5 % bis 5 Gew.-% beträgt; bezogen auf die Gesamtmasse der Zusammensetzung, oder wobei die Zusammensetzung umfasst: eine Verbindung ATV014 und einen pharmazeutisch annehmbaren Hilfsstoff, wobei der pharmazeutisch annehmbare Hilfsstoff mindestens einen ausgewählt aus einem Verdünnungsmittel, einem Zerfallsmittel, einem Bindemittel, einem Schmiermittel und weiteren Zusatzmaterialien umfasst, wobei das Verdünnungsmittel mindestens eines ausgewählt aus mikrokristalliner Cellulose und Mannitol umfasst, wobei das Zerfallsmittel ein aus Croscarmellose-Natrium ausgewähltes Zerfallsmittel umfasst, das Bindemittel ein aus Hydroxypropylcellulose ausgewähltes Bindemittel umfasst, und das Schmiermittel mindestens eines ausgewählt aus Magnesiumstearat, Stearinsäure und Natriumstearylfumarat umfasst, wobei, jeweils der Gehalt der Verbindung ATV014 50 Gew.-% bis 60 Gew.-% beträgt, der Gehalt des Verdünnungsmittels 30 Gew.-% bis 40 Gew.-% beträgt, der Gehalt des Zerfallsmittels 2 Gew.-% bis 5 Gew.-% beträgt, der Gehalt des Bindemittels 2 Gew.-% bis 5 Gew.-% beträgt und der Gehalt des Schmiermittels 1,5 % bis 5 Gew.-% beträgt; bezogen auf die Gesamtmasse der Zusammensetzung, oder wobei die Zusammensetzung umfasst: eine Verbindung ATV014 und einen pharmazeutisch annehmbaren Hilfsstoff, wobei der pharmazeutisch annehmbare Hilfsstoff mindestens einen ausgewählt aus einem Verdünnungsmittel, einem Zerfallsmittel, einem Bindemittel, einem Schmiermittel und weiteren Zusatzmaterialien umfasst, wobei das Verdünnungsmittel mikrokristalline Cellulose PH102 oder Mannitol 50C ist, wobei das Zerfallsmittel ein aus Croscarmellose-Natrium ausgewähltes Zerfallsmittel umfasst, das Bindemittel ein aus Hydroxypropylcellulose ausgewähltes Bindemittel umfasst, und das Schmiermittel mindestens eines ausgewählt aus Magnesiumstearat, Stearinsäure und Natriumstearylfumarat umfasst, wobei, jeweils der Gehalt der Verbindung ATV014 50 Gew.-% bis 60 Gew.-% beträgt, der Gehalt des Verdünnungsmittels 30 Gew.-% bis 40 Gew.-% beträgt, der Gehalt des Zerfallsmittels 2 Gew.-% bis 5 Gew.-% beträgt, der Gehalt des Bindemittels 2 Gew.-% bis 5 Gew.-% beträgt und der Gehalt des Schmiermittels 1,5 % bis 5 Gew.-% betragen kann; bezogen auf die Gesamtmasse der Zusammensetzung, oder
wobei die Zusammensetzung umfasst: eine Verbindung ATV014 und einen pharmazeutisch annehmbaren Hilfsstoff, wobei der pharmazeutisch annehmbare Hilfsstoff mindestens einen ausgewählt aus einem Verdünnungsmittel, einem Zerfallsmittel, einem Bindemittel, einem Schmiermittel und weiteren Zusatzmaterialien umfasst, wobei das Verdünnungsmittel mikrokristalline Cellulose PH102 und Mannitol 50C ist, wobei das Zerfallsmittel ein aus Croscarmellose-Natrium ausgewähltes Zerfallsmittel umfasst, das Bindemittel ein aus Hydroxypropylcellulose ausgewähltes Bindemittel umfasst, und das Schmiermittel mindestens eines ausgewählt aus Magnesiumstearat, Stearinsäure und Natriumstearylfumarat umfasst, wobei, jeweils der Gehalt der Verbindung ATV014 50 Gew.-% bis 60 Gew.-% beträgt, der Gehalt des Verdünnungsmittels 30 Gew.-% bis 40 Gew.-% beträgt, der Gehalt des Zerfallsmittels 2 Gew.-% bis 5 Gew.-% beträgt, der Gehalt des Bindemittels 2 Gew.-% bis 5 Gew.-% beträgt und der Gehalt des Schmiermittels 1,5 % bis 5 Gew.-% beträgt; bezogen auf die Gesamtmasse der Zusammensetzung, oder
wobei die Zusammensetzung umfasst: eine Verbindung ATV014 und einen pharmazeutisch annehmbaren Hilfsstoff, wobei der pharmazeutisch annehmbare Hilfsstoff mindestens einen ausgewählt aus einem Verdünnungsmittel, einem Zerfallsmittel, einem Bindemittel, einem Schmiermittel und weiteren Zusatzmaterialien umfasst, wobei das Verdünnungsmittel Mannitol 50C ist, wobei das Zerfallsmittel ein aus Croscarmellose-Natrium ausgewähltes Zerfallsmittel umfasst, das Bindemittel ein aus Hydroxypropylcellulose ausgewähltes Bindemittel umfasst, und das Schmiermittel mindestens eines ausgewählt aus Magnesiumstearat, Stearinsäure und Natriumstearylfumarat umfasst, wobei, jeweils der Gehalt der Verbindung ATV014 50 Gew.-% bis 60 Gew.-% beträgt, der Gehalt des Verdünnungsmittels 30 Gew.-% bis 40 Gew.-% beträgt, der Gehalt des Zerfallsmittels 2 Gew.-% bis 5 Gew.-% beträgt, der Gehalt des Bindemittels 2 Gew.-% bis 5 Gew.-% beträgt und der Gehalt des Schmiermittels 1,5 % bis 5 Gew.-% beträgt; bezogen auf die Gesamtmasse der Zusammensetzung.

6. Zusammensetzung nach einem der Ansprüche 1 bis 3, wobei die Dosierungsform der Zusammensetzung eine feste orale Dosierungsform oder eine Injektion ist;
gegebenenfalls ist die Dosierungsform eine Tablette, ein Granulat oder eine Kapsel.

7. Verwendung der Zusammensetzung nach einem der Ansprüche 1 bis 6 bei der Zubereitung eines Produkts zur Vorbeugung, Linderung oder Behandlung einer Coronavirus-Infektion oder der Replikation oder Ausbreitung eines homologen Variantenvirus davon und einer daraus resultierenden zytopathischen Wirkung oder eines Produkts zum Erkennen eines Coronavirus oder eines homologen Variantenvirus davon.

8. Verwendung nach Anspruch 7, wobei die Infektion Fieber, Husten, Halsschmerzen, Pneumonie, akute Atemwegsinfektion, schwere akute Atemwegsinfektion, hyperoxische Ateminsuffizienz und akutes Atemnot-Syndrom, Sepsis oder septischen Schock umfasst.

9. Verwendung nach einem der Ansprüche 7 bis 8, wobei das Coronavirus umfasst: MHV-A59, HCoV-229E, HCoV-OC43, HCoV-NL63, HCoV-HKU1, SARS-CoV, MERS-CoV, SARS-CoV-2, ein Virus der Maushepatitis, ein Virus der infektiösen Katzenperitonitis, ein Coronavirus des Hundes, ein Coronavirus des Rindes, ein Virus der infektiösen Bronchitis der Vögel oder ein Coronavirus des Schweins; vorzugsweise umfasst das SARS-CoV-2 einen Mutantenstamm oder einen Nicht-Mutantenstamm von SARS-CoV-2; noch bevorzugter umfasst der Mutantenstamm von SARS-CoV-2 den SARS-CoV-2-Mutantenstamm B.1, den SARS-CoV-2-Mutantenstamm B.1.351, den SARS-CoV-2-Mutantenstamm B.1.617.2, den SARS-CoV-2-Mutantenstamm C.37, den SARS-CoV-2-Mutantenstamm P.1-Linie, den SARS-CoV-2-Mutantenstamm B.1.525, den SARS-CoV-2-Mutantenstamm B.1.427 oder den SARS-CoV-2-Mutantenstamm B.1.429.

10. Verwendung nach einem der Ansprüche 7 bis 8, wobei die Zusammensetzung geeignet ist zur Verwendung bei Menschen oder Tieren; und/oder das Tier Rinder, Pferde, Schafe, Schweine, Hunde, Katzen, Nagetiere, Primaten, Vögel oder Fische umfasst.

11. Verfahren zur Zubereitung der Zusammensetzung nach einem der Ansprüche 1 bis 6, umfassend: Mischen der Verbindung ATV014 und des pharmazeutisch annehmbaren Hilfsstoffs, Durchführen einer Trockengranulation, Zugeben eines externen Schmiermittels, Mischen und Tablettieren oder Kapselabfüllen oder Verpacken, um die Zusammensetzung zu erhalten; oder
Mischen der Verbindung ATV014 und des pharmazeutisch annehmbaren Hilfsstoffs, Durchführen einer Nassgranulation, Mahlen, Trocknen, Trockenmahlen, Zugeben eines externen Schmiermittels, Mischen und Tablettieren oder Kapselabfüllen oder Verpacken, um die Zusammensetzung zu erhalten; oder
Mahlen oder Pulverisieren der Verbindung ATV014, Mischen der Verbindung und des pharmazeutisch annehmbaren Hilfsstoffs, Durchführen einer Trockengranulation, Zugeben eines externen Schmiermittels, Mischen und Tablettieren oder Kapselabfüllen oder Verpacken, um die Zusammensetzung zu erhalten; oder
Mahlen oder Pulverisieren der Verbindung ATV014, Mischen der Verbindung und des pharmazeutisch annehmbaren Hilfsstoffs, Durchführen einer Nassgranulation, Mahlen, Trocknen, Trockenmahlen, Zugeben eines externen Schmiermittels, Mischen und Tablettieren oder Kapselabfüllen oder Verpacken, um die Zusammensetzung zu erhalten.

## Revendications

1. Composition, comprenant : un composé ATV014 et un excipient pharmaceutiquement acceptable, 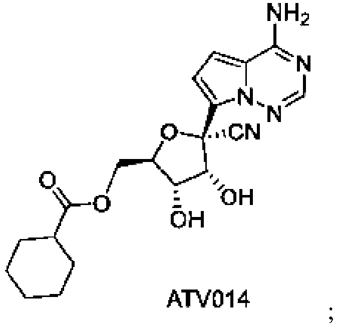 dans laquelle les excipients pharmaceutiquement acceptables comprennent au moins un élément choisi parmi un diluant, un désagrégeant, un liant et un lubrifiant, et dans laquelle la composition ne comprend pas de tensioactif.

2. Composition selon la revendication 1, dans laquelle l'excipient pharmaceutiquement acceptable comprend d'autres matières auxiliaires.

3. Composition selon la revendication 2, dans laquelle le diluant comprend au moins un élément choisi parmi la cellulose microcristalline, le mannitol et l'amidon prégélatinisé ; de préférence, le diluant est la cellulose microcristalline ou le mannitol ; plus préférentiellement, le diluant est la cellulose microcristalline PH102 ou le mannitol 50C ; le plus préférentiellement, le diluant est le mannitol 50C ; et/ou
le désagrégeant comprend au moins un élément choisi parmi la croscarmellose sodique, la crospovidone, le glycolate d'amidon sodique et l'hydroxypropylcellulose ; et/ou
le liant comprend au moins un élément choisi parmi l'hydroxypropylcellulose, la povidone et l'amidon ; et/ou
le lubrifiant comprend au moins un élément choisi parmi le stéarate de magnésium, l'acide stéarique et le fumarate de stéaryle de sodium ; et/ou
les autres matériaux auxiliaires comprennent un matériau auxiliaire choisi parmi un glissant ; et/ou
le glissant comprend un glissant choisi parmi le dioxyde de silicium colloïdal ou le talc ; et/ou
la composition comprend en outre un lubrifiant externe ; et/ou
le lubrifiant externe comprend au moins un élément choisi parmi le stéarate de magnésium, le stéarate de sodium et le fumarate de stéaryle de sodium.

4. Composition selon l'une quelconque des revendications 2 à 3, dans laquelle la teneur en composé ATV014 est comprise entre 15 % en poids et 70 % en poids, par rapport à la masse totale de la composition ; et/ou
la teneur en diluant est comprise entre 20 % en poids et 70 % en poids, par rapport à la masse totale de la composition ; et/ou
la teneur en désagrégeant est comprise entre 1 % en poids et 10 % en poids, par rapport à la masse totale de la composition ; et/ou
la teneur en liant est comprise entre 1 % en poids et 10 % en poids, par rapport à la masse totale de la composition ; et/ou
la teneur en lubrifiant est comprise entre 0,5 % en poids et 5 % en poids, par rapport à la masse totale de la composition ; et/ou
la teneur en lubrifiant externe est comprise entre 0,5 % en poids et 5 % en poids, par rapport à la masse totale de la composition ; et/ou
la composition ne comprend pas de laurylsulfate de sodium.

5. Composition selon l'une quelconque des revendications 2 à 3, comprenant : un composé ATV014 et un excipient pharmaceutiquement acceptable, l'excipient pharmaceutiquement acceptable comprenant au moins un élément choisi parmi un diluant, un désagrégeant, un liant, un lubrifiant, et d'autres matériaux auxiliaires, le diluant comprenant au moins un élément choisi parmi la cellulose microcristalline, le mannitol, l'hydrophosphate de calcium et l'amidon prégélatinisé, le désagrégeant comprenant au moins un élément choisi parmi la croscarmellose sodique, la crospovidone, le glycolate d'amidon sodique et l'hydroxypropylcellulose, le liant comprenant au moins un élément choisi parmi l'hydroxypropylcellulose, la povidone et l'amidon, et le lubrifiant comprenant au moins un élément choisi parmi le stéarate de magnésium, l'acide stéarique et le fumarate de stéaryle de sodium, dans laquelle, par rapport à la masse totale de la composition, la teneur en composé ATV014 est comprise entre 15 % en poids et 70 % en poids, la teneur en diluant est comprise entre 20 % en poids et 70 % en poids, la teneur en désagrégeant est comprise entre 1 % en poids et 10 % en poids, la teneur en liant est comprise entre 1 % en poids et 10 % en poids, et la teneur en lubrifiant est comprise entre 0,5 % en poids et 5 % en poids ; ou
comprenant : un composé ATV014 et un excipient pharmaceutiquement acceptable, l'excipient pharmaceutiquement acceptable comprenant au moins un élément choisi parmi un diluant, un désagrégeant, un liant, un lubrifiant, et d'autres matériaux auxiliaires, le diluant comprenant au moins un élément choisi parmi la cellulose microcristalline, le mannitol, l'hydrophosphate de calcium et l'amidon prégélatinisé, le désagrégeant comprenant au moins un élément choisi parmi la croscarmellose sodique, la crospovidone, le glycolate d'amidon sodique et l'hydroxypropylcellulose, le liant comprenant au moins un élément choisi parmi l'hydroxypropylcellulose, la povidone et l'amidon, et le lubrifiant comprenant au moins un élément choisi parmi le stéarate de magnésium, l'acide stéarique et le fumarate de stéaryle de sodium, dans laquelle, par rapport à la masse totale de la composition, la teneur en composé ATV014 est comprise entre 50 % en poids et 60 % en poids, la teneur en diluant est comprise entre 30 % en poids et 40 % en poids, la teneur en désagrégeant est comprise entre 2 % en poids et 5 % en poids, la teneur en liant est comprise entre 2 % en poids et 5 % en poids, et la teneur en lubrifiant est comprise entre 1,5 % en poids et 5 % en poids ; ou
comprenant : un composé ATV014 et un excipient pharmaceutiquement acceptable, l'excipient pharmaceutiquement acceptable comprenant au moins un élément choisi parmi un diluant, un désagrégeant, un liant, un lubrifiant, et d'autres matériaux auxiliaires, le diluant comprenant au moins un élément choisi parmi la cellulose microcristalline et le mannitol, le désagrégeant comprenant un désagrégeant choisi parmi la croscarmellose sodique, le liant comprenant un liant choisi parmi l'hydroxypropylcellulose, et le lubrifiant comprenant au moins un élément choisi parmi le stéarate de magnésium, l'acide stéarique et le fumarate de stéaryle de sodium, dans laquelle, par rapport à la masse totale de la composition, la teneur en composé ATV014 est comprise entre 50 % en poids et 60 % en poids, la teneur en diluant est comprise entre 30 % en poids et 40 % en poids, la teneur en désagrégeant est comprise entre 2 % en poids et 5 % en poids, la teneur en liant est comprise entre 2 % en poids et 5 % en poids, et la teneur en lubrifiant est comprise entre 1,5 % en poids et 5 % en poids ; ou
comprenant : un composé ATV014 et un excipient pharmaceutiquement acceptable, l'excipient pharmaceutiquement acceptable comprenant au moins un élément choisi parmi un diluant, un désagrégeant, un liant, un lubrifiant, et d'autres matériaux auxiliaires, le diluant étant la cellulose microcristalline PH102 ou le mannitol 50C, le désagrégeant comprenant un désagrégeant choisi parmi la croscarmellose sodique, le liant comprenant un liant choisi parmi l'hydroxypropylcellulose, et le lubrifiant comprenant au moins un élément choisi parmi le stéarate de magnésium, l'acide stéarique et le fumarate de stéaryle de sodium, dans laquelle, par rapport à la masse totale de la composition, la teneur en composé ATV014 est comprise entre 50 % en poids et 60 % en poids, la teneur en diluant est comprise entre 30 % en poids et 40 % en poids, la teneur en désagrégeant est comprise entre 2 % en poids et 5 % en poids, la teneur en liant est comprise entre 2 % en poids et 5 % en poids, et la teneur en lubrifiant peut être comprise entre 1,5 % en poids et 5 % en poids ; ou
comprenant : un composé ATV014 et un excipient pharmaceutiquement acceptable, l'excipient pharmaceutiquement acceptable comprenant au moins un élément choisi parmi un diluant, un désagrégeant, un liant, un lubrifiant, et d'autres matériaux auxiliaires, le diluant étant la cellulose microcristalline PH102 et le mannitol 50C, le désagrégeant comprenant un désagrégeant choisi parmi la croscarmellose sodique, le liant comprenant un liant choisi parmi l'hydroxypropylcellulose, et le lubrifiant comprenant au moins un élément choisi parmi le stéarate de magnésium, l'acide stéarique et le fumarate de stéaryle de sodium, dans laquelle, par rapport à la masse totale de la composition, la teneur en composé ATV014 est comprise entre 50 % en poids et 60 % en poids, la teneur en diluant est comprise entre 30 % en poids et 40 % en poids, la teneur en désagrégeant est comprise entre 2 % en poids et 5 % en poids, la teneur en liant est comprise entre 2 % en poids et 5 % en poids, et la teneur en lubrifiant est comprise entre 1,5 % en poids et 5 % en poids ; ou
comprenant : un composé ATV014 et un excipient pharmaceutiquement acceptable, l'excipient pharmaceutiquement acceptable comprenant au moins un élément choisi parmi un diluant, un désagrégeant, un liant, un lubrifiant, et d'autres matériaux auxiliaires, le diluant étant le mannitol 50C, le désagrégeant comprenant un désagrégeant choisi parmi la croscarmellose sodique, le liant comprenant un liant choisi parmi l'hydroxypropylcellulose, et le lubrifiant comprenant au moins un élément choisi parmi le stéarate de magnésium, l'acide stéarique et le fumarate de stéaryle de sodium, dans laquelle, par rapport à la masse totale de la composition, la teneur en composé ATV014 est comprise entre 50 % en poids et 60 % en poids, la teneur en diluant est comprise entre 30 % en poids et 40 % en poids, la teneur en désagrégeant est comprise entre 2 % en poids et 5 % en poids, la teneur en liant est comprise entre 2 % en poids et 5 % en poids, et la teneur en lubrifiant peut être comprise entre 1,5 % en poids et 5 % en poids.

6. Composition selon l'une quelconque des revendications 1 à 3, dans laquelle la forme posologique de la composition est une forme posologique orale solide ou une injection ;
optionnellement, la forme posologique est un comprimé, un granulé ou une capsule.

7. Utilisation de la composition selon l'une quelconque des revendications 1 à 6 dans la préparation d'un produit destiné à prévenir, soulager ou traiter une infection par un coronavirus, ou la réplication ou la propagation d'un virus variant homologue de celui-ci, et un effet cytopathique généré à partir de celui-ci, ou un produit destiné à détecter un coronavirus ou un virus variant homologue de celui-ci.

8. Utilisation selon la revendication 7, dans laquelle l'infection comprend de la fièvre, une toux, un mal de gorge, une pneumonie, une infection respiratoire aiguë, une infection respiratoire aiguë sévère, une insuffisance respiratoire hypoxique et un syndrome de détresse respiratoire aiguë, une septicémie ou un choc septique.

9. Utilisation selon l'une quelconque des revendications 7 à 8, dans laquelle le coronavirus comprend : MHV-A59, HCoV-229E, HCoV-OC43, HCoV-NL63, HCoV-HKU1, SARS-CoV, MERS-CoV, SARS-CoV-2, un virus de l'hépatite de la souris , un virus de la péritonite infectieuse féline, un coronavirus canin, un coronavirus bovin, un virus de la bronchite infectieuse aviaire ou un coronavirus porcin ; de préférence, le SARS-CoV-2 comprend une souche mutante ou une souche non mutante du SARS-CoV-2 ; plus préférentiellement, la souche mutante du SARS-CoV-2 comprend une souche mutante B.1 du SARS-CoV-2, une souche mutante B.1.351 du SARS-CoV-2, une souche mutante B.1.617.2 du SARS-CoV-2, une souche mutante C.37 du SARS-CoV-2, une lignée de souche mutante P.1 du SARS-CoV-2, une souche mutante B.1.525 du SARS-CoV-2, une souche mutante B.1.427 du SARS-CoV-2 ou une souche mutante B.l.429 du SARS-CoV-2.

10. Utilisation selon l'une quelconque des revendications 7 à 8, dans laquelle la composition est adaptée à une utilisation chez l'homme ou l'animal ; et/ou
l'animal comprend un animal bovin, équin, ovin, porcin, canin, félin, rongeur, primate, aviaire ou piscicole.

11. Procédé de préparation de la composition selon l'une quelconque des revendications 1 à 6, comprenant : le mélange du composé ATV014 et de l'excipient pharmaceutiquement acceptable, la réalisation d'une granulation par voie sèche, l'ajout d'un lubrifiant externe, le mélange, et la compression en comprimés ou le remplissage de capsules ou le conditionnement afin d'obtenir la composition ; ou
le mélange du composé ATV014 et de l'excipient pharmaceutiquement acceptable, la réalisation d'une granulation par voie humide, le broyage, le séchage, le broyage à sec, l'ajout d'un lubrifiant externe, le mélange et la compression en comprimés ou le remplissage de capsules ou le conditionnement afin d'obtenir la composition ; ou
le broyage ou la pulvérisation du composé ATV014, le mélange du composé et de l'excipient pharmaceutiquement acceptable, la réalisation d'une granulation par voie sèche, l'ajout d'un lubrifiant externe, le mélange, et la compression en comprimés ou le remplissage de capsules ou le conditionnement afin d'obtenir la composition ; ou
le broyage ou la pulvérisation du composé ATV014, le mélange du composé et de l'excipient pharmaceutiquement acceptable, la réalisation d'une granulation par voie humide, le broyage, le séchage, le broyage à sec, l'ajout d'un lubrifiant externe, le mélange et la compression en comprimés ou le remplissage de capsules ou le conditionnement afin d'obtenir la composition.
